# EUROPEAN PATENT APPLICATION

(11) **EP 1 669 457 A1**
(43) Date of publication of application: **14.06.2006**
(21) Application number: 04788280.8
(22) Date of filing: 29.09.2004
(51) Int. Cl.: C12P 7/24, C07C 29/141, C07C 31/20, C07C 45/65, C07C 47/22, C07C 51/235, C07C 59/01, C07C 67/39, C07C 69/653, C12P 7/18, C12P 7/40, C12P 7/42, C12P 7/62, C07B 61/00

(54) **PROCESS FOR PRODUCING 3-HYDROXYPROPIONALDEHYDE**

(30) Priority: 29.09.2003 JP 2003337663
(71) Applicant: NIPPON SHOKUBAI CO., LTD., Osaka-shi, Osaka 541-0043 (JP)
(72) Inventor: TORAYA, Tetsuo, 7000002 (JP); TOBIMATSU, Takamasa;, Okayama-shi,Okayama 7000927 (JP); YAMANISHI, Mamoru, 68512 (US); MORI, Kouichi, 7191102 (JP); KAJIURA, Hideki, Amagasaki-shi, Hyogo 661-0001 (JP); YAMADA, Seiki, Ibaraki 302-0100 (JP); YUZUKI, Michio;, Chiba 272-0001 (JP); AZUMA, Muneaki, 7011212 (JP); HARA, Tetsuya, Osaka 5600055 (JP); YASUDA, Shinzo, Ibaraki 300-1221 (JP)
(74) Representative: Bassil, Nicholas Charles
(86) International application number: PCT/JP2004/014213
(87) International publication number: WO 2005/030972

(57) **Abstract**

A process in which 3-hydroxypropionaldehyde can be produced from glycerin at high conversion rate. This process is **characterized by** including the step of dehydrating glycerin with bacterial cells and/or bacterial cell treatment product containing diol dehydratase and/or glycerol dehydratase optionally together with a diol dehydratase reactivating factor and/or glycerol dehydratase reactivating factor under such conditions that the quotient of catalytic amount [X (U/g glycerin)] of diol dehydratase and/or glycerol dehydratase divided by square of glycerin concentration [Y (g/100 ml)], X/Y², is in the range of 10 to 8,000 so as to obtain 3-hydroxypropionaldehyde.

## Description

### Technical Field

The present invention relates to a method for producing 3-hydroxypropionaldehyde and a method for producing 1,3-propanediol, 3-hydroxypropionic acid, acrolein, acrylic acid and an acrylic ester produced from 3-hydroxypropionaldehyde produced by the method. In particular, the present invention relates to a method which can efficiently produce 3-hydroxypropionaldehyde in high purity and a method which can efficiently produce 1,3-propanediol, 3-hydroxypropionic acid, acrolein, acrylic acid and an acrylic ester in high purity from the 3-hydroxypropionaldehyde.

### Background Art

1, 3-Propanediol is a useful compound having a wide range of applications, for example, as a monomer used for producing polyester and polyurethane, and a starting material for synthesizing a cyclic compound.

As a method for synthesizing 1,3-propanediol, both of a method by chemical synthesis and a method by fermentation have been well-known. As the former method, for example, a method for producing 1,3-propanediol via carbonylation of ethylene oxide using a rhodium catalyst (for example, US-A-4,873,378, US-A-873,379 and US-A-4,935,554), and a method for producing 1,3-propanediol by reducing 3-hydroxypropionaldehyde (for example, US-A-2,434,110) have been known.

However, the method by the chemical synthesis is not sufficient in conversion ratio and selectivity, and not favorable in the viewpoint of cost, because a purification process is required to remove a by-product. In addition, if 3-hydroxypropionaldehyde as a raw material contains a by-product, a secondary product might be further formed in the subsequent production process of 1,3-propanediol, and this could require a difficult purification, or cause discoloration or undesirable polymerization in products such as fibers in a production of textiles using this 1,3-propanediol as a raw material thereafter. For this reason, a content of by-product in 3-hydroxyprpionaldehyde to be used as a raw material for producing 1, 3-propanediol is desirably as low as possible.

Further, the latter method is a method of producing 1,3-propanediol through the fermentation of glycerin or glucose using a strain producing 1,3-propanediol such as Citrobacter, Clostridium, Enterobacter, Ilyobacter, Klebsiella, Lactobacillus and Pelobacter, and the like. This method comprises two-step reaction consisting of a step of converting glycerin to 3-hydroxypropionaldehyde (3-HPA) and water with a dehydratase and a step of reducing the resultant 3-HPA tol,3-propanediolwith a NAD+-link-oxidoreductase. In addition, to improve yield of desired 1,3-propanediol, a method for producing 1,3-propanediol from glycerin using a recombinant microorganism has been disclosed (for example, WO 98/21339, WO 99/58686, US-A-6,025,184 and WO 01/12833).

However, in the production method by the fermentation, it has been known that, to obtain NADH necessary for the latter reaction among the reactions, the two-step reaction and a reaction to form NADH by dehydrogenation to dihydroxyacetone occur simultaneously. By this reason, there is a problem that a conversion ratio from glycerin to 1,3-propanediol by the general fermentation becomes as low as around 50% resulting in an insufficient yield of 3-HPA. To solve this problem, production of 1,3-propanediol using a recombinant microorganism has been reported. However, even if such microorganism is used, since a reaction to form NADH needs to occur in addition to the reaction from glycerin to 3-HPA by the similar reason to the above, it is very difficult to attain a high conversion ratio. Further, a fermentation culture generally contains many by-products such as nutrients contained in the culture and microbial products. For this reason, the method using fermentation is also not preferable from an economical viewpoint, because a purification process becomes complicated much more than that by chemical synthesis. In addition to the problem, there is another problem that the method by the fermentation often uses an organic solvent such as cyclohexane in a purification process of the desired product, 1,3-propanediol, and further needs post-treatment of such organic solvent considering the environment.

On the other hand, 3-hydroxypropionaldehyde, which is an intermediate in the method, can be used also as an intermediate in the production of 3-hydroxypropionic acid, acrolein, acrylic acid and acrylic esters, as well as 1,3-propanediol as described above. Among these products, acrylic acid, for example, has a wide range of applications, and has been used as a copolymer for acrylic fiber, adhesives or agglutinant in emulsion, as well as coating materials, textile processing, leather, construction materials, and the like. Production method for acrylic acid has been conventionally known, and it is generally produced by a two-step gas phase catalytic oxidation, that is, from propylene to acrolein and from acrolein to acrylic acid. In this case, acrolein used as an intermediate material can be also produced via treatment of 3-hydroxypropionaldehyde under acidic conditions. Accordingly, to produce acrylic acid in high purity and at low cost, it is desirable that acrolein and further 3-hydroxypropionaldehyde are efficiently produced in high purity.

As a method for producing 3-hydroxypropionaldehyde (3-HPA), a method for producing 3-HPA from glycerol using a recombinant strain obtained by cloning of diol dehydratase and/or glycerol dehydratase in combination with diol dehydratase reactivating factor and/or glycerol dehydratase reactivating factor (for example, Journal of Bacteriology, Vol.181, No.13, pp.4110-4113, 1999; The Journal of Biological Chemistry, Vol.272, No.51, pp.32034-32041, 1997; Arch. Microbiol., 174:81-88(2000); The Journal of Biological Chemistry, Vol. 274, No.6, pp. 3372-3377, 1999), and a method for converting glycerin to 3-HPA by culturing Klebsiella pneumoniae in a glycerin-rich culture medium followed by suspending in a buffer containing semicarbazide and glycerin (Applied and Environmental Microbiology, Vol. 50, No.6, pp.1444-1450, 1985) have been reported. Among the methods, however, the method for producing 3-HPA from glycerin using a recombinant strain is, in any case, a method to scholastically study a behavior in an initial stage of the reaction, and specific reaction conditions of the conversion from the glycerin to 3-HPA need more study from the industrial viewpoint. Further, in the latter method, though a yield of around 83% at most can be attained due to an accumulation of 3-HPA facilitated by the presence of a semicarbazide, when 3-HPA is recovered in the presence of the semicarbazide, 3-HPA forms a complex with the semicarbazide as shown in the following reaction scheme, from which 3-HPA cannot be recovered again. Thus, the method cannot provide 3-HPA as a simple substance.

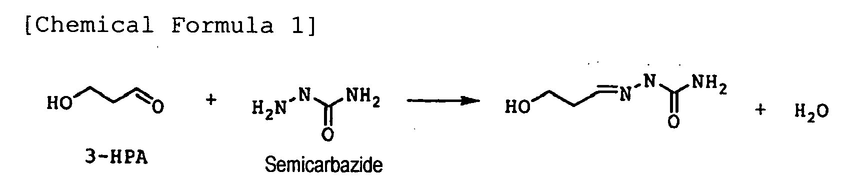

### Disclosure of the Invention

### Problems to be Solved by the Invention

Thus, an object of the present invention is to provide specific conditions for producing 3-hydroxypropionaldehyde (3-HPA) in industrially high conversion ratio (that is, in high yield).

Another object of the present invention is to provide a method for producing 1,3-propanediol from the 3-HPA efficiently.

Further another object of the present invention is to provide a method for producing 3-hydroxypropionaldehyde acid from the 3-HPA efficiently.

Still further another object of the present invention is to provide a method for producing acrolein, acrylic acid or an acrylic ester from the 3-HPA in high purity and in high yield.

### Means to Solve the Problems

It has been generally well-known that activity (amount) of enzyme to act as a catalyst and concentration of substrate greatly influence conversion ratio. The present inventors have, after extensively studying a method for converting glycerin to 3-HPA to attain the objects in consideration of the circumstances, obtained such knowledge that there is a very strong correlation between catalytic activity and substrate concentration, in particular, in the conversion. Further, based on this knowledge, the present inventors have, after extensively studying a conversion from glycerin to 3-HPA using a microbial cell, toluene-treated microbial cell or immobilized microbial cell having diol dehydratase and/or glycerol dehydratase (herein collectively referred to as "diol/glycerol dehydratase" or simply as "dehydratase") and diol dehydratase reactivating factor and/or glycerol dehydratase reactivating factor (herein collectively referred to as "diol/glycerol dehydratase reactivating factor" or simply as "dehydratase reactivating factor"), found that by applying an action of dehydratase with an amount of catalyst controlled so as to give a value (X/Y²) calculated by dividing a catalytic amount [X (U/g glycerin)] by square of glycerin concentration [Y (g/100 ml) within a specified range, a conversion ratio in a high level corresponding to the industrial level, in particular, a conversion ratio not less than 70% can be attained, and 3-HPA can be produced in a high yield. Still further, the present inventors have also found that since almost all components other than glycerin such as nutrients are not used, particularly when a treated microbial cell is used, 3-hydroxypropionaldehyde can be easily produced in high purity only by removing the treated microbial cells by filtration or the like. Furthermore, the present inventors have obtained such knowledge that by hydrogenation of the thus obtained 3-hydroxypropionaldehyde, desired 1,3-propanediol can be produced in high purity and in high yield.

Further, the present inventors have also obtained such knowledge that acrolein can be obtained by reacting 3-hydroxypropionaldehyde scarcely containing by-products obtained as described above under acidic conditions, and this acrolein can be produced in high purity because the starting material scarcely contains any by-product, hence, from this acrolein, acrylic acid can be produced in high purity. In addition, the present inventors found that an acrylic ester can be also produced in high purity simply by the oxidative esterification of acrolein.

Namely, the object can be attained by a method for producing 3-hydroxypropionaldehyde which comprises a step of dehydrating glycerin using a microbial cell and/or a treated microbial cell containing diol dehydratase and/or glycerol dehydratase, and optionally diol dehydratase reactivating factor and/or glyceroldehydratase reactivating factor, under conditions so as to give a value (X/Y²) calculated by dividing a catalytic amount [X (U/g glycerin)] of diol dehydratase and/or glycerol dehydratase by square of glycerin concentration [Y (g/100 ml)] within a range of 10 to 8,000, to produce 3-hydroxypropionaldehyde.

The another object can be attained by a method for producing 1, 3-propanediol comprising a step of hydrogenating 3-hydroxypropionaldehyde produced by the method to produce 1,3-propanediol.

The further another object can be attained by a method for producing 3-hydroxypropionic acid comprising a step of oxidizing 3-hydroxypropionaldehyde produced by the method to produce 3-hydroxypropionic acid.

The still further another object can be attained by a method for producing acrolein comprising a step of removing microbial cell and/or treatedmicrobial cell from the reaction product containing 3-hydroxypropionaldehyde produced by the method, followed by reacting 3-hydroxypropionaldehyde under acidic conditions to produce acrolein; a method for producing acrylic acid comprising a step of oxidizing acrolein produced by the method to produce acrylic acid; and a method for producing an acrylic ester comprising a step of subjecting acrolein produced by the method to oxidative esterification to produce acrylic ester.

### Best Mode for Carrying Out the Invention

The present invention will be explained in detail below.

A first aspect of the present invention relates to a method for producing 3-hydroxypropionaldehyde which comprises a step of dehydrating glycerin using a microbial cell and/or a treated microbial cell containing diol dehydratase and/or glycerol dehydratase, and optionally diol dehydratase reactivating factor and/or glycerol dehydratase reactivating factor, under conditions so as to give a value (X/Y²) calculated by dividing a catalytic amount [X (U/g glycerin)] of diol dehydratase and/or glycerol dehydratase by square of glycerin concentration [Y (g/100 ml)] within a range of 10 to 8, 000, to produce 3-hydroxypropionaldehyde. This means that among important factors considered to affect on a conversion ratio from glycerin to 3-HPA, in particular, an amount of catalyst greatly affects on a conversion ratio, and it has been revealed that by acting dehydratase on glycerin in such an amount that a value (X/Y²) calculated by dividing a catalytic amount [X (U/g glycerin)] of diol dehydratase and/or glycerol dehydratase by square of glycerin concentration [Y (g/100 ml)] is in a specified range as of 10 to 8, 000, glycerin is selectively utilized in a conversion to 3-HPA without an occurrence of undesirable reaction such as a conversion of glycerin to NADH, and glycerin can be converted to 3-HPA in such a high conversion ratio as not less than 80%. Further, since conversion from glycerin to 3-HPA occurs preferentially and 3-hydroxypropionaldehyde to be produced scarcely contains a by-product, 3-hydroxypropionaldehyde can be produced in high purity by such a simple procedure as to separate / remove microbial cell / treated microbial cell.

In the present invention, a value (X/Y²) calculated by dividing a catalytic amount [X (U/g glycerin)] of diol dehydratase and/or glycerol dehydratase by square of glycerin concentration [Y (g/100 ml)] is in the range of 10 to 8,000. In this case, when X/Y² is fallen in this range, a high conversion ratio, for example, a conversion ratio of not less than 70% can be attained. On the contrary, when the X/Y² deviates from the range, the conversion ratio from glycerin to 3-HPA remarkably decreases. Lower limit of the X/Y ² is preferably 7,000, 6,500, 5,500 and 5,000 in this order.

As used herein, "glycerin concentration [Y (g/100 ml)]" represents a weight of glycerin contained in 100 ml of a solution. For example, since 1 L of 50 mM potassium phosphate buffer (pH 8) containing 1 M of glycerin contains 92 g of glycerin, a glycerin concentration (Y) of this case becomes 9.2 (g/100 ml).

Further, as used herein, "catalytic amount [X (U/g glycerin)] " represents a value of a unit (U) of diol / glycerol dehydratase enzyme divided by a weight (g) of glycerin. In this case, "1U" as a unit of enzyme means an ability of microbial cell and/or treated microbial cell exhibiting an activity of diol/glycerol dehydratase enzyme to convert 1 µmol of glycerin to 3-HPA per 1 minute, corresponds to an ability of microbial cell and/or treated microbial cell exhibiting a diol/glycerol dehydratase enzyme activity to convert 1 µmol of 1, 2-propanediol to propionaldehyde per 1 minute. In this connection, in the present invention, the formations of propionaldehyde and 3-hydroxypropionaldehyde were detected by a detection method using 3-methyl-2-benzothiazolinonehydrazone hydrochloride. For example, when a toluene-treated microbial cell having an activity of 200 U is added to 1 L of 50 mM potassium phosphate buffer (pH 8) containing 1 M of glycerin, a catalytic amount (X) becomes 2.17 (U/g glycerin) (= 200/92).

In the present invention, glycerin concentration (Y) is not especially limited, and may be selected, as appropriate, depending on viscosity of reaction liquid, amount of enzyme to be added, kind and intensity of enzymatic activity, concentration and purification processes after reaction. Lower limit of glycerin concentration (Y) is preferably not less than 0.5 g, more preferably not less than 1 g, and most preferably not less than 2 g, per 100 ml of solution. Also, upper limit of glycerin concentration (Y) is preferably not more than 60 g, more preferably not more than 50 g, and most preferably not more than 40 g, per 100 ml of solution. In this case, when glycerin concentration is less than 0.5 g/100 ml, due to too low glycerin amount in a solution, an amount of 3-HPA to be formed would become unduly low, and the concentration could be not economical from the industrial viewpoint. On the contrary, when glycerin concentration is over 60 g / 100 ml, a viscosity of reaction liquid would increase thereby making homogeneous mixing with microbial cell / treated microbial cell difficult, glycerin could be impossible to efficiently receive an action of diol dehydratase / glycerol dehydratase.

In the present invention, dehydrataze including diol dehydratase and glycerol dehydratase is coenzyme B12 dependent type as described above, and presence of coenzyme B12 is inevitable to convert glycerin to 3-HPA. Amount of coenzyme B12 to be present in the conversion from glycerin to 3-HPA is not especially limited so long as the conversion from glycerin to 3-HPA proceeds sufficiently thereby, and differs depending on concentration of substrate and the like. An amount of coenzyme B12 to be present is preferably in the range of 1 to 1,000 µM, more preferably 10 to 800 µM in a concentration of coenzyme B12 per 50 mM of substrate concentration. Further, amount of microbial cell and/or treated microbial cell is not especially limited so long as the catalytic amount of enzyme satisfies the relation: X/Y² = 10 to 8,000, and a conversion of glycerin to 3-HPA sufficiently proceeds therewith, and differs depending on form of microbial cell and/or treated microbial cell to be used (microbial cell, immobilized microbial cell and immobilized factor) and source thereof, concentration of substrate (glycerin) and volume of reaction liquid. For example, microbial cell and/or treated microbial cell may be used in a batch type or in a flow reaction. When immobilized microbial cell is used in a flow reaction, an amount of microbial cell and/or treated microbial cell differs depending on kind of microorganism to be used, life of enzyme, flow rate of reaction liquid (LHSV) and the like. Also, an amount of microbial cell and/or treated microbial cell is not especially limited so long as the conversion of glycerin to 3-HPA sufficiently proceeds therewith. It usually about 10 to 100 times of amount as compared to that of batch type is used. Lower limit of catalytic amount (X) of enzyme, although be easily calculated from the value of X/Y², is preferably not less than 2.5 U/g glycerin, for example. Similarly, upper limit of catalytic amount (X) of enzyme can be also easily calculated from the value of X/Y², suitable value of Y and the like, but it is preferably not more than 27,000,000 U. In this case, when the catalytic amount of enzyme is less than 2.5 U/g glycerin, an action of enzyme to glycerin could be insufficient, and yield of desired 3-HPA could be also insufficient. On the contrary, even when a catalytic amount is over 28,800,000 U/g glycerin, effects obtained by amount of enzyme added could fail to be obtained which is not preferable from the economical viewpoint. In this connection, as for unit of the enzyme, an ability of microbial cell and/or treated microbial cell exhibiting diol/glycerol dehydratase enzymatic activity to convert 1 µmol of 1,2-propanediol to propionaldehyde is defined as "1 U", and measuring method thereof is not especially limited, and any method can be used so long as formation of propionaldehyde from 1, 2-propanediol can be detected thereby. In the present invention, formations of propionaldehyde and 3-hydroxypropionaldehyde were detected by a method to detect using 3-methyl-2-benzothiazolinone hydrochloride.

In the present invention, "diol/glycerol dehydratase" or "dehydratase" is an enzyme having a catalytic action to convert glycerin to 3-hydroxypropionaldehyde (herein also referred to as "3-HPA") and water by dehydration. Any known enzyme can be used without special limitation so long as the enzyme has such an action. Among these enzymes, in view of life of enzyme, glycerol dehydratase is preferably used.

In the present invention, glycerol dehydratase is not especially limited, and glycerol dehydratase derived from any source having / expressing this enzyme may be used. Specifically, these include microorganisms belonging to Klebsiella genus, Citrobacter genus, Clostridium genus, Lactobacillus genus, Enterobacter genus, Caloramator genus, Salmonella genus and Listeria genus, and more specifically, glycerol dehydratase derived from Klebsiella pneumoniae, Citrobacter pneumoniae, Clostridium Pasteurianum, Lactobacillus leichmannii, Citrobacter intermedium, Lactobacillus reuteri, Lactobacillus buchneri, Lactobacillus brevis, Enterobacter agglomerans, Clostridium butyricum, Caloramator viterbensis, Lactobacillus collinoides, Lactobacillus hilgardii, Salmonella typhimurium, Listeria monocytogenes and Listeria innocua. These glycerol dehydratases may be used alone or in combination of two or more kinds, or may be used in combination with diol dehydratase described in detail below.

Method for isolating glycerol dehydratase from the sources is not especially limited, and glycerol dehydratase may be isolated from any microorganism as described above similarly to a known separation and isolation method such as extraction and column chromatography.

Further, in the present invention, diol dehydratase is also not especially limited. Any diol dehydratase derived from any source having / expressing this enzyme may be used. Specifically, these include microorganisms belonging to Klebsiella genus, Propionibacterium genus,Clostridium genus, Lactobacillus genus, Salmonella genus, Citrobacter genus, Flavobacterium genys, Acetobacterium genus, Brucella genus and Fusobacterium genus, and more specifically, diol dehydratase derived from Klebsiella pneumoniae, Propionibacterium freudenreichii, Clostridium glycolicum, Lactobacillus brevis, Salmonella typhimurium, Citrobacter freundii, Lactobacillus buchneri, Brucella melitensis, Fusobacterium nucleatum, Klebsiella oxytoca, Salmonella typhimurium, Listeria monocytogenes and Listeria innocua. These diol dehydratases may be used alone or in combination of two or more kinds, or may be used in combination with glycerol dehydratase described in detail above.

Method for isolating diol dehydratase from the sources is not especially limited, and diol dehydratase may be isolated from any microorganism as described above similarly to a known separation and isolation method of enzyme such as extraction and column chromatography.

Microbial cell and /or treated microbial cell may contain, in addition to diol/glycerol dehydratase, diol/glycerol dehydratase reactivating factor. In this case, in view of conversion ratio of glycerin to 3-HPA and reactivation of enzyme, microbial cell / treated microbial cell having diol/glycerol dehydratase reactivating factor is preferably used.

In the present invention, "diol/glycerol dehydratase reactivating factor" or "dehydratase reactivating factor" is referred to as a factor to induce again (reactivate) an activity of dehydratase, which has been deactivated by catalyzing a reaction of glycerin to 3-HPA + H₂0. In more detail, the coenzyme B12 is involved in the reaction of glycerin to 3-HPA + H₂O, which is catalyzed by dehydratase, and active center of the dehydratase is deactivated after developing and catalyzing the reaction of glycerin to 3-HPA + H₂0 due to collapse of the coenzyme B12. Here, the dehydratase reactivating factor replaces the collapsed coenzyme B12 with a new coenzyme B12 to induce again (reactivate) an activity of the dehydratase and make the dehydratase reusable for dehydration reaction of glycerin. Thus, in the present invention, a factor having an ability to reactivate the dehydratase is referred to as "diol/glycerol dehydratase reactivating factor" or "dehydratase reactivating factor". The dehydratase reactivating factor is not especially limited so long as the factor has an ability as described above, and well-known glycerol dehydratase reactivating factor which activates deactivated glycerol dehydratase and diol dehydratase reactivating factor which activates a deactivated diol dehydratase can be used. In thisconnection,dehydratase and dehydratase reactivating factor may be used in any combination of those described above. Dehydratase reactivating factor is preferably composed of a large subunit of diol dehydratase reactivating factor and/or glycerol dehydratase reactivating factor and a small subunit of diol dehydratase reactivating factor and/or glycerol dehydratase reactivating factor, more preferably composed of a large subunit of diol dehydratase reactivating factor and a small subunit of diol dehydratase reactivating factor and/or glycerol dehydratase reactivating factor, and particularly preferably composed of a large subunit of diol dehydratase reactivating factor and a small subunit of diol dehydratase reactivating factor. In this case, a large subunit of diol dehydratase reactivating factor includes, for example, ddrA (NCBI No. AF017781) and a large subunit of glycerol dehydratase reactivating factor includes, for example, gdrA (NCBI No. U30903), but not limited to these. A small subunit of diol dehydratase reactivating factor includes, for example, ddrB (NCBI No. AF017781) and a small subunit of glycerol dehydratase reactivating factor includes, for example, gdrB (NCBI No. U30903), but not limited to these. These reactivating factors may be used alone or as a mixture of two or more kinds. Further, order of a large subunit and a small subunit is not especially limited, and may be in any order of a large subunit and a small subunit, or a small subunit and a large subunit, or, when each of these units exists in plural, these units may exist in any of block or random, but from the viewpoint of a high reactivation ability, preferably a large subunit exist in upstream of a small subunit.

In the present invention, a source of the glycerol dehydratase reactivating factor is not especially limited. The factor is coded on a genome of a microorganism having glycerol dehydratase as described above, and composed of a large subunit and a small subunit. Specifically, glycerol dehydratase reactivating factors derived from microorganisms belonging to Lactobacillus genus, Klebsiella genus, Citrobacter genus, Clostridium genus and Enterobacter genus, and more specifically, the microorganisms include, for example, Lactobacillus sp., Klebsiella pneumoniae, Lactobacillus leichmannii, Citrobacter freundii, Citrobacter intermedium, Lactobacillus reuteri, Lactobacillus buchneri, Lactobacillus brevis, Clostridium Pasteurianum, Enterobacter agglomerans and Clostridium butyricum may be cited. Among these microorganisms, microorganisms belonging to Lactobacillus genus, for example, Lactobacillussp., Lactobacillusleichmannii, Lactobacillus reuteri, Lactobacillus buchneri and Lactobacillus brevis may be preferably used, and glycerol dehydratase reactivating factors derived from Lactobacillus sp. and Lactobacillus reuteri are particularly preferably used. In this connection, sources of glycerol dehydratase and glycerol dehydratase reactivating factor may be the same or different from each other.

Method for isolating glycerol dehydratase reactivating factor from the sources is not especially limited, and glycerol dehydratase reactivating factor may be isolated from any microorganism as described above similarly to a well-known separation and isolation method of enzyme such as extraction and column chromatography.

Further, in the present invention, source of the diol dehydratase reactivating factor is not especially limited. The factor is coded on a genome of a microgranism having diol dehydratase as described above, and composed of a large subunit and a small subunit. In this connection, this glycerol dehydratase reactivating factor is preferably used because the factor can reactivate both of glycerol dehydratase and diol dehydratase. Specifically, diol dehydratase reactivating factors derived from microorganisms belonging to Klebsiella genus, Citrobacter genus, Propionibacterium genus, Lactobacillus genus, Flavobacterium genus and Acetobacterium genus may be used. More specifically, the microorganisms include, for example, Klebsiella pneumoniae, Citrobacter freundii, Propionibacterium freudenreichii, Lactobacillus brevis, Lactobacillus buchneri, Flavobacterium sp. and Acetobacterium sp. Among these microrganims, diol dehydratase reactivating factors derived from microorganisms belonging to Lactobacillus genus, that is, Lactobacillus brevis and Lactobacillus buchneri, in particular, Lactobacillus brevis can be preferably used. In this connection, sources of diol dehydratase and diol dehydratase reactivating factor may be the same or different from each other, but from the viewpoint of more superior reactivation ability, a large subunit of diol dehydratase reactivating factor is preferably used. Accordingly, the combination of a large subunit of diol dehydratase reactivating factor and a small subunit of glycerol dehydratase reactivating factor and/or diol dehydratase reactivating factor is more preferably used.

Method for isolating diol dehydratase reactivating factor from the sources is not especially limited, and glycerol dehydratase reactivating factor may be isolated from any microorganism as described above similarly to a well-known separation and isolation method of enzyme such as extraction and column chromatography.

Alternatively, a recombinant microorganism containing a gene coding for dehydratase and/or dehydratase reactivating factor and a microorganismmutated so as to improve an activity of dehydratase and/or dehydratase reactivating factor may be used as a microorganism source having/expressing the dehydratase and/or dehydratase reactivating factor. These recombinant microorganisms can be prepared using a conventionalmethod. For example,microorganismsexpressing dehydrataseinclude,specifically,those disclosedin Journal of Bacteriology, Vol.181, No.13, pp.4110-4113, 1999; The Journal of Biological Chemistry, Vol.272, No.51, pp.32034-32041, 1997; Arch. Microbiol., 174:81-88 (2000); The Journal of Biological Chemistry, Vol.274, No.6, pp. 3372-3377, 1999, all of which were described in the section of Description of Related Art, as well as those disclosed in WO 98/21339, WO 99/58686, WO 98/21341, US-A-6,025,184, WO 01/12833, WO 96/35795, WO 01/04324, FEMS Microbiology Letters 164(1998) 21-28, Applied and Environmental Microbiology, Jan. 1998, p. 98-105, Applied and Environmental Microbiology, Dec. 1991, p.3541-3546, and the like. Also, microorganisms mutated so as to improve an activity of dehydratase and/or dehydratase reactivating factor can be prepared using a conventional method, and include, specifically, for example, those disclosed in WO 00/70057 and the like.

In the present invention, 3-hydroxypropionaldehyde can be produced by dehydration of glycerin using microbial cell and/or treated microbial cell containing diol/glycerol dehydratase and, if necessary, diol/glycerol dehydratase reactivating factor. In this case, from the viewpoints that a very high conversion ratio can be attained as described later in detail, and 3-hydroxypropionaldehyde obtained contains less amount of by-products, preferably "microbial cell" and "treated microbial cell" do not use glycerin as an energy source of the microorganism. In the present invention, treated microbial cell is more preferably used. This is because use of treated microbial cell allows conversion of glycerin to 3-HPA by a method other than fermentation and thus attainment of high conversion ratio and low content of by-product.

In this connection, in the present invention, "microbial cell" is not especially limited so long as the microbial cell has diol dehydratase and/or glycerol dehydratase, and, if necessary, diol dehydratase reactivating factor and/or glycerol dehydratase reactivating factor, and an ability to convert glycerin to 3-HPA. It does not matter whether a microbial cell has a proliferating ability or not when the microbial cell is returned to a suitable culture medium after completion of the reaction of the present invention. Preferably, microbial cell is one in which 3-HPA is not further used to another reaction, and which allows conversion of glycerin to 3-HPA in a reaction system containing glycerin by a method other than fermentation, that is, does not assimilate glycerin nor proliferate using glycerin as an energy source. For example, these microbial cells include preferably a microbial cell obtained by culturing a microbial cell having an ability to convert glycerin to 3-HPA under aerobic conditions; a microbial cell obtained by culturing the microbial cell under conditions so as not to produce NADH and/or NADPH; and a recombinant microbial cell obtained by introducing diol/glycerol dehydratase or diol/glycerol dehydratase reactivating factor to a suitable host, and more preferably a microbial cell which has been genetically manipulated so as to have a plurality of copies of diol/glycerol dehydratase to highly express these enzymes and the like. Further, "fermentation" means an activity of microorganism performing, in a reaction system in which 3-hydroxypropionaldehyde is obtained from glycerin, at a same time, conversion of glycerin and/or proliferation using glycerin as an energy source and/or oxidation of glycerin and the like. Hence, in the present invention, "method other than fermentation" means a reaction method in which 3-hydroxypropionaldehyde is formed by dehydration of glycerin in a reaction system containing glycerin of the present invention, but at the same time not being accompanied by conversion of glycerin, proliferation using glycerin as an energy and oxidation of glycerin.

Herein, "treated microbial cell" means a microbial cell which has been treated so as to be easily used for the reaction of the present invention. Specifically, examples of the treated microbial cell include, for example, immobilized substance (for example, immobilized enzyme) such as immobilized dehydratase or immobilized dehydratase reactivating factor as described above; toluene-treated microbial cell which is obtained by treating microbial cell containing dehydratase or dehydratase reactivating factor as described above with an organic solvent such as toluene; and immobilized microbial cell of a microbial cell having dehydratase or dehydratase reactivating factor as described above. Among these, toluene-treated microbial cell and immobilized microbial cell are preferably used, and toluene-treated microbial cell is particularly preferable. Immobilizing method in producing a immobilized factor is not especially limited, and well-known methods such as a method by which a factor is immobilized on an insoluble substrate via covalent bond, ionic bond, adsorption or the like; a method by which a factor is cross-linked each other; and a method by which a factor is included in a network structure of a polymer, may be used.

Also, immobilizing method for producing an immobilized microbial cell is not especially limited, and conventional methods such as a method by which microorganism having dehydratase or dehydratase reactivating factor as described above is supported on an insoluble substrate; a method by which the microorganism is immobilized by being absorbed or included in a gel matrix; and a method by which the microorganism is entrapped in an internal space, may be used.

Further, production method for the toluene-treated microbial cell is also not especially limited. Conventional methods such as a method which comprises adding toluene to a microorganism having dehydratase or dehydratase reactivating factor as described above and stirring the mixture, thereby forming holes having such a size that an enzyme and a factor can not go out from the microbial cell may be used. In the method, though toluene was used to produce a toluene-treated microbial cell, another organic solvent such as acetone, hexane and ethyl acetate may be used instead of toluene. Among these, toluene is preferably used. In this case, amount of the organic solvent to be added is preferably in the range of 0.1 to 10% by mass, and more preferably 0.2 to 5% by mass. Further, the toluene-treated microbial cell is not especially limited, and can be prepared by conventional methods. For example, the following method can be used: A microbial cells cultured under suitable conditions are suspended in a buffer, and toluene is added to this suspension so as to give an adequate concentration, preferably a final concentration of 1% (v/v), then the suspension is vigorously stirred for a prescribed time, preferably about 5 minutes using a vortex mixer or the like to perform toluene treatment, subsequently the microbial cell after the toluene treatment is washed with a buffer. In the method, buffer is not especially limited, and well-known buffers can be used. 50 mM potassium phosphate buffer (25 ml of 1 M K₂HPO₄ and 100 ml of 1 M K₂HPO₄ are mixed, pH is adjusted at 8, then water is added to 25 ml of the solution to make 500 ml as a whole) is particularly preferably used.

In the present invention, immobilized microbial cell and immobilized factormaybe used in any form, and specifically, used, for example, in a form of membrane-like, particulate, ribbon-like and folded layer-like. In view of easiness in handling, ribbon-like and particle-like are preferably used.

By using microbial cell and/or treated microbial cell, stabilization of microbial cell and/or treated microbial cell containing dehydratase and dehydratase reactivating factor can be performed. In particular, when treated microbial cell is used, in addition to an advantage that these microbial cell can be continuously and repeatedly used, another advantage exists that purity and yield of the desired 3-hydroxypropionaldehyde can be improved because glycerin can be conveniently converted to 3-hydroxypropionaldehyde without using a culture medium.

In the present invention, method for converting glycerin to 3-HPA is not especially limited, and a method similar to conventionally known methods can be used using a factor, an immobilized microbial cell and an immobilized factor. Differing from the case of fermentation, since the conversion of glycerin to 3-HPA according to the present invention is carried out using microbial cell and/or treated microbial cell (for example, toluene-treated microbial cell and immobilized microbial cell), 3-hydroxypropionaldehyde to be obtained scarcely contains by-product, and further, since almost all of glycerin is used for the reaction, a high conversion ratio can be attained. Further, in comparing with conventional chemical method, since raw material is mainly glycerin, and both the conversion ratio and selectivity to 3-hydroxypropionaldehyde are very high and purity is also high, a purification process to remove by-product can be performed conveniently, and the method is very advantageous from the economical viewpoint.

A preferable embodiment of the conversion of glycerin to 3-HPA according to the present invention will be described below. For example, when a particulate immobilizedmicrobial cell and/or immobilized factor is used, it is added into a mixed solution containing a suitable buffer (for example, potassium phosphate buffer), an appropriate amount of coenzyme B12 as described above and glycerin, so that a enzyme as described above is suitably present, and then the resultant mixture is stirred at 10 to 90°C, preferably at 15 to 85°C for 1 to 360 minutes, preferably for 5 to 120 minutes to form 3-HPA. The 3-HPA thus formed is in a mixed state with immobilized microbial cell on the particle, and can be easily separated from the immobilized microbial cell by a conventional method such as filtration, ultrafiltration and settling. Alternatively, 3-HPA may be formed by passing a mixed solution containing a suitable buffer (for example, potassium phosphate buffer), an appropriate amount of coenzyme B12 as described above, glycerin and the like through a column packed with a particulate immobilized microbial cell and/or immobilized factor at 10 to 90°C, preferably at 15 to 85°C, and at a flow rate of 0.1 to 50 LHSV, preferably 0.2 to 40 LHSV. In this case, glycerin concentration is not especially limited, so long as the factor can sufficiently act thereon, and it is varied with a catalytic amount of diol dehydratase and/or glycerol dehydratase, and also may be such a concentration at which X/Y² is not fallen within a range of 10 to 8,000. Preferably, it is in the range of 0.1 to 50% (w/v), more preferably 0.2 to 40% (w/v). Further, in the method, liquid used for dissolving glycerin is not especially limited so long as glycerin can be dissolved. Water, and various buffers such as physiological saline, potassium phosphate buffer,potassium citrate buffer,phosphate buffer, Good's buffer and Tris buffer may be used, for example. Among these, water, potassium phosphate buffer and phosphate buffer are preferably used. In the present invention, presence of potassium ion in a reaction system is preferable in view of an activity of enzyme. For the presence of potassium ion in the reaction system, buffers containing potassium salts such as potassium phosphate buffer, potassium citrate buffer and other potassium salt aqueous solution is particularly preferably used as a reaction medium. Concentration of potassium ion is not especially limited, but preferably in the range of 5 mM to 1 M, and more preferably 10 to 500 mM.

According to the method of the present invention, the 3-HPA thus obtained is, after microbial cell and/or treated microbial cell being removed, hydrogenated to form desired 1,3-propanediol. Thus, a second aspect of the present invention relates to a method for producing 1,3-propanediol which comprises a step of removing the microbial cell and/or treated microbial cell from the 3-hydroxypropionaldehyde produced by the method of the present invention, subsequently hydrogenating said 3-hydroxypropionaldehyde to produce 1,3-propanediol. According to the method of the present invention, since the conversion from glycerin to 3-HPA occurs preferentially in a high conversion ratio, 3-propionaldehyde of the product can be produced in a high purity without containing not only glycerin as a substrate but also any by-product. Accordingly, by using 3-HPA of the present invention, a high purity of 3-HPA can be obtained by a simple procedures to separate or remove microbial cell / treated microbial cell. Further, by hydrogenating this 3-HPA, 1,3-propanediol can also be produced in a high purity.

In the present invention, separation / removal of microbial cell is not especially limited, and performed by a conventional method. Specifically, by using a known method such as filtration, ultrafiltration and settling, immobilized microbial cell can be easily separated from 3-HPA.

In the present invention, hydrogenation may be performed by any of fermentation or chemical synthesis method, but chemical synthesis method is preferable. This is because there is an advantage that by producing 1, 3-propanediol from 3-HPA using chemical synthesis method, purity and yield of desired 1,3-propanediol can be improved. In this case, in the chemical synthesis method, conventional hydrogenation method may be used, and the reaction may be carried out either in a gas phase or a liquid phase. 3-HPA is hydrogenated preferably in a liquid phase, and more preferably in an aqueous solution, to form desired 1,3-propanediol. The chemical synthesis method to produce 1,3-propanediol from 3-HPA is not especially limited, and conventional methods can be used. For example, the methods include, for example, a method which comprises adding palladium carbon to 3-HPA, replacing a gas phase part with hydrogen, and hydrogenating the 3-HPA with hydrogen while stirring; a method which comprises hydrogenating 3-HPA in the presence of a heterogeneous catalyst having ruthenium supported on an oxide carrier at a temperature of 30 to 180°C and under a hydrogen pressure of 5 to 300 bar, in an aqueous solution of pH value 2.5 to 7.0 (for example, JP-A-2002-516614) ; a method which comprises hydrogenating 5 to 100% by weight of 3-HPA in an aqueous solution, on a catalyst having platinum supported on titanium oxide as a carrier, at a temperature of 30 to 180°C, at pH value of 2.5 to 6.5, and under a hydrogen pressure of 5 to 300 bar (for example, JP-A-5-213800); and a method which comprises catalytically hydrogenating 3-HPA in an aqueous solution in the presence of a catalyst such as a catalyst having Pt supported on TiO₂ and a Ni/Al₂O₃/SiO₂ - catalyst, under a hydrogen pressure of 5 to 300 bar, at pH value of 2.5 to 6.5, and at a temperature of 30 to 180°C (for example, JP-A-6-40973).

According to the method of the present invention, 1,3-propanediol and water are mainly formed, and other by-products and secondary products are scarcely present. Hence, purification consists mainly of removal of water only, not complicated, and when fiber is produced using this 1,3-propanediol as a raw material thereafter, there is no risk resulting discoloration and defective polymerization in a product such as fiber. In this case, purification method of 1, 3-propanediol to be used is not especially limited, and conventional methods can be used. For example, a method such as by distillation and reverse osmosis membrane can be used.

According to the present invention, by oxidizing 3-hydroxypropionaldehyde obtained similarly as above, 3-hydroxypropionic acid is produced. Thus, a third aspect of the present invention relates to a method which comprises a step of oxidizing the 3-hydroxypropionaldehyde produced by the method of the present invention to produce 3-hydroxypropionic acid.

In the present invention, in view of purity of 3-hydroxypropionic acid, microbial cell / treated microbial cell has been preferably separated or removed in advance prior to oxidation of 3-hydroxypropionaldehyde. In this case, separation / removal of microbial cell / treated microbial cell is not especially limited, but may be performed by conventional methods. Specifically, by using known methods suchasfiltration, ultrafiltration and settling, immobilized microbial cell can be removed from 3-HPA.

In the present invention, although production of 3-hydroxypropionic acid from 3-HPA may be performed by any of fermentation or chemical synthesis method, in view of purity and yield of 3-hydroxypropionic acid to be produced, chemical synthesis method is preferably used. In the oxidation according to the present invention, conventional oxidation methods can be used, and the reaction may be carried out either in a gas phase or a liquid phase. 3-HPA is oxidized preferably in a liquid phase, and more preferably in an aqueous solution. Method to be used in this case is not especially limited, conventional methods such as a method using platinum carbon, palladium carbon, or the like can be used. Specifically, the method includes a method which comprises adding palladium carbon to 3-HPA, and replacing a gas phase part with hydrogen, and oxidizing the 3-HPA with oxygen while stirring; and a method which comprises adding platinum carbon and sodium bicarbonate as catalysts to 3-HPA reaction liquid and contacting the 3-HPA with oxygen to oxidize the 3-HPA.

According to the method of the present invention, 3-hydroxypropionic acid can be mainly formed. For this reason, purification is easy. In this case, purification is not necessarily required. Purification method for 3-hydroxypropionic acid before use is not especially limited, and conventional methods, for example, a method such as distillation and reverse osmosis membrane can be used.

Further, according to the present invention, through a reaction of 3-hydroxypropionaldehyde obtained similarly as described above under acidic conditions, acrolein can be produced. Thus, a fourth aspect of the present invention relates to a method for producing acrolein which comprises a step of reacting the 3-hydroxypropionaldehyde produced by the method of the present invention under acidic conditions, to produce acrolein. Further, according to the present invention, through an oxidation of acrolein obtained by the fourth aspect of the present invention, acrylic acid is produced. Thus, a fifth aspect of the present invention relates to a method for producing acrylic acid which comprises a step of oxidizing the acrolein produced by the method of the present invention to produce acrylic acid.

In the fourth and fifth aspects of the present invention, in view of purities of acrolein and acrylic acid as final products,microbial cell / treated microbial cell has been preferably separated or removed in advance prior to oxidation of 3-hydroxypropionaldehyde. In this case, separation / removal of microbial cell / treated microbial cell is not especially limited, but may be performed by conventional methods. Specifically, by using known methods such as filtration, ultrafiltration and settling, immobilized microbial cell can be removed from 3-HPA.

In the present invention, production of acrolein from 3-HPA can be easily attained through a reaction of 3-HPA under acidic conditions. For example, acrolein can be efficiently produced by adding / mixing an acid such as hydrochloric acid, sulfuric acid and nitric acid, preferably hydrochloric acid, to a solution containing 3-HPA so as to give a pH value of 1 to 5, preferably 1.5 to 4.5, then standing at 5 to 90°C, preferably at 7 to 85°C for 1 to 360 minutes, preferably 2 to 120 minutes. Since no components other than acid are used in this process, by-product is scarcely formed. Since the reaction has a high conversion ratio and a high selectivity, acrolein can be produced in a high purity and in a high yield.

In the present invention, acrolein thus formed is oxidized to produce acrylic acid. Production method from acrolein to acrylic acid is not especially limited, and may be any of fermentation or chemical synthesis method. However, in view of purity and yield of acrylic acid to be produced, chemical synthesis method is preferably used. In the oxidation according to the present invention, conventional oxidation methods can be used, and the reaction may be carried out either in a gas phase or a liquid phase. Further, oxidation method for acrolein in liquid phase is not also especially limited, and conventional methods can be used. For example, a method for producing acrylic acid by oxidizing acrolein in the presence of a palladium carbon catalyst while oxygen is added to reaction liquid containing acrolein, can be used. Further, oxidation method for acrolein in gas phase is not especially limited, and conventional methods can be used. For example, known methods such as disclosed in JP-A-64-63543 and JP-A-63-146841 can be used. Specifically, catalyst to be used for oxidizing acrolein to acrylic acid is not especially limited, and conventional catalysts can be used alone or in combination thereof. For example, catalysts containing molybdenum and vanadium, and preferably a catalyst represented by the general formula: MOₐ-V_{b}-W_{c}-Cu_{d}-Aₑ-B_{f}-C_{g}-Oₓ (wherein Mo represents molybdenum, V represents vanadium, W represents tungsten, Cu represents copper, A represents at least one element selected among antimony, bismuth, tin, niobium, cobalt, iron, nickel and chromium, B represents at least one element selected among alkali metals and alkaline earth metals, C represents at least one element selected from silicon, aluminum, zirconium and titanium, O represents oxygen, each of a, b, c, d, e, f, g and x represents atomic ratio of Mo, V, W, Cu, A, B, C and O, respectively, provided that when a = 12, then b = 2 to 14, c = 0 to 12, d = 0.1 to 5, e = 0 to 5, f = 0 to 5, g = 0 to 20, and x is a value depending on oxidation state of each element) may be cited. Further, preparation method and mixing and molding method of catalyst to be used in this case are also not especially limited, and methods and raw materials which have been generally used in the art can be employed. Further, form of catalyst is also not especially limited, and for example, spherical, cylindrical and column-shaped forms can be used. As for forming method, support forming, extrusion forming, tablet compression, and the like can be used, and still further, a catalyst having these catalyst materials supported on a refractory carrier is also useful.

Further, reaction conditions used in the conversion from acrolein to acrylic acid is also not especially limited. For example, the reaction is performed by mixing acrolein with oxygen needed for conversion to acrylic acid and steam, and feeding this acrolein-containing gas under a reaction pressure in a range of atmospheric to 0.5 MPa, at a space velocity in a range of 300 to 5, 000 hr⁻¹ (STP), with a reaction temperature being controlled at 200 to 400°C, and preferably 220 to 380°C.

The acrilic acid thus obtained is recovered by a common method. For example, the acrylic acid forming gas is, after cooled with a heat exchanger, made in countercurrent contact with a catching solvent containing a polymerization inhibitor to obtain acrylic acid aqueous solution, from which acrylic acid is isolated by a method such as extraction, distillation and azeotropic distillation.

Further, in the present invention, acrolein obtained in the fourth aspect is then converted to acrylic esters via oxidative esterification in the presence of a catalyst. Thus, a sixth aspect of the present invention relates to a method for producing an acrylic ester which comprises a step of subjecting the acrolein produced by the method of the present invention, to produce an acrylic ester. In this case, production method of an acrylic ester from acrolein is not especially limited, and any of fermentation or chemical synthesis method can be used. However, in view of purity and yield of an acrylic ester to be produced, chemical synthesis method is preferably used. In the oxidation according to the present invention, conventional oxidation methods can be used, and the reaction may be carried out either in a gas phase or a liquid phase. The reaction is performed particularly preferably in a liquid phase in the presence of a catalyst. In this case, as a catalyst, the similar catalyst as used in the production of acrylic acid can be used. Further, conditions of reaction from acrolein to acrylic ester is also not especially limited, and for example, the similar conditions as used in the production of acrylic acid can be used. The thus obtained acrylic ester is recovered by a common method.

### EXAMPLES

The present invention will be explained more specifically with referring to working examples below.

### Example 1

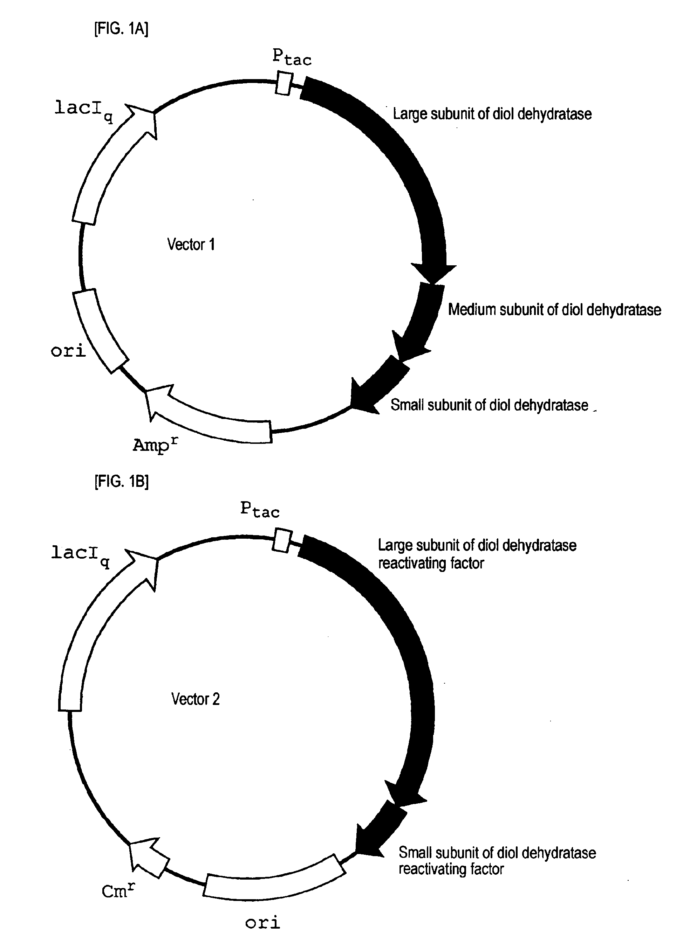

A strain JM109/vector 1 (DD) /vector 2 (DDR), which was obtained by transforming E. coli JM 109 as a host with vector 1 (Fig. 1A, SEQ ID NO: 1) obtained by inserting a gene coding for diol dehydratase of Klebsiella pneumoniae (ATCC 25955) into a plasmid having a replication origin (ori) derived from pBR322 and vector 2 (Fig. 1B, SEQ ID N0: 2) obtained by inserting a gene coding for diol dehydratase reactivating factor of Klebsiella pneumoniae (ATCC 25955) into a plasmid having a replication origin (ori) derived from p15A, was inoculated into a LB medium containing 50 µg/ml of ampicillin and 100 µg/ml of chloramphenicol, and cultured at 37°C for 15 hours. The cultured liquid was inoculated into 200 ml of a LB medium containing 50 pg/ml of ampicillin and 100 µg/ml of chloramphenicol, and cultured with shaking at 37°C. When an absorbance at 660 nm reached 0.8 (OD = 0.8) after initiation of the culture, IPTG was added so as to give a concentration of 1 mM, and cultured for further 5 hours, then the culture was stopped. Microbial cells were collected by centrifugation. Collected cells were washed twice with 50 mM of potassium phosphate buffer (pH 8), and suspended in 50 mM potassium phosphate buffer (pH 8) so as to give OD660 = 0.2. This microbial cell suspending fluid was used as a crude enzyme fluid. SEQ ID NO: 1 and SEQ ID NO: 2 show base sequences of vector 1 and vector 2 used in the present Example.

To 4 ml of the crude enzyme fluid, 3 ml of 50 mM potassium phosphate buffer (pH 8), 1 ml of 2M glycerin, 1 ml of 0.5 M KC1 and 1 ml of 150 µM coenzyme B12 were added, and the mixture was reacted at 37°C for 60 minutes. An aliquot of the reaction liquid was taken and this aliquot is tested for quantitative determination of 3-hydroxypropionaldehyde therein. An equivalent amount of 0.1 M potassium citrate buffer (pH 3.0) was added to the reaction liquid to stop the reaction. An equivalent amount of water was added thereto, and 0.5 times by volume of an aqueous solution of 0.1% 3-methyl-2-benzothiazolinon hydrazone hydrochloride monohydrate was added, absorbance at 305 nm was measured to determine a concentration of 3-hydroxypropionaldehyde. The result indicated that 0.1 M 3-hydroxypropionaldehyde was formed in the reaction mixture. The residual reaction mixture was filtered to remove microbial cells. After the filtrate was placed into a test tube with a sealed cap, 0.015 g of 5% palladium carbon was added thereto, and a gas phase part was replaced with hydrogen gas. A balloon was filled with 500 ml of hydrogen gas under normal pressure, connected to the gas phase part and sealed, and the reaction was carried out with stirring at 60°C for 5 hours in a hot-water bath. Analysis of the reaction mixture indicated that 0.098 M of 1, 3-propandiol was formed. In this case, catalyst amount of microbial cells [X (U/g glycerin)], concentration of glycerin [Y (g/100 ml) ], and X/Y² are summarized in Table 1 below. In Table 1, the values of [X (U/g glycerin)], [Y (g/100 ml)], and X/Y² disclosed in the documents listed as related references in the section of Description of Related Art are also summarized together in Table 1 below.

### Example 2

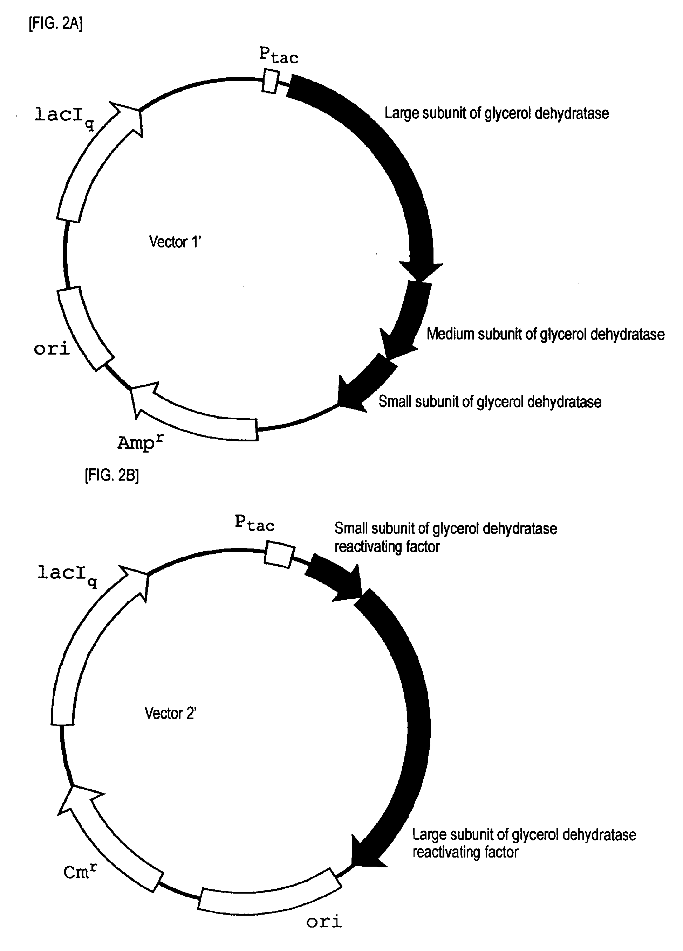

A strain JM109/vector 1' (GD)/vector 2' (GDR), which was obtained by transforming E. coli JM 109 as a host with vector 1' (Fig. 2A, SEQ ID NO: 3) obtained by inserting a gene coding for glycerol dehydratase of Klebsiella pneumoniae (ATCC 25955) into a plasmid having replication origin (ori) derived from pBR322 and vector 2' (Fig. 2B, SEQ ID NO: 4) obtained by inserting a gene coding for glycerol dehydratase reactivating factor of Klebsiella pneumoniae (ATCC 25955) into a plasmid having replication origin (ori) derived from p15A, was inoculated into a LB medium containing 50 µg/ml of ampicillin and 100 µg/ml of chloramphenicol, and cultured at 37°C for 15 hours. The cultured liquid was inoculated into 200 ml of a LB medium containing 50 µg/ml of ampicillin and 100 µg/ml of chloramphenicol, and cultured with shaking at 37°C. When an absorbance at 660 nm reached 0.8 (OD = 0.8) after initiation of the culture, IPTG was added so as to give a concentration of 1 mM, and cultured for further 5 hours, then the culture was stopped. Microbial cells were collected by centrifugation. Collected cells were washed twice with 50 mM of potassium phosphate buffer (pH 8), and suspended in 50 mM potassium phosphate buffer (pH 8) so as to give OD660 = 0.2. This microbial cell suspending fluid was used as a crude enzyme fluid. Toluene was added to the suspension so as to give a final concentration of 1%. After the mixture was stirred by using a vortex mixer for 5 minutes, microbial cells were collected by centrifugation. Microbial cells were suspended into 50 mM potassium phosphate buffer (pH 8) so as to give OD660 of 0.2. This suspension was designated as a toluene-treated microbial cell fluid. In this connection, SEQ ID NO: 3 and SEQ ID NO: 4 show base sequences of vector 1' and vector 2' used in the present Example.

To the toluene-treated microbial cell fluid, 3 ml of 50 mM potassium phosphate buffer (pH 8), 1 ml of 2M glycerin, 1 ml of 0.5 M KCl and 1 ml of 150 uM coenzyme B12 were added, and the mixture was reacted at 37°C for 20 minutes. An aliquot of the reaction liquid was taken and this aliquot is tested for quantitative determination of 3-hydroxypropionaldehyde therein. An equivalent amount of 0.1 M potassium citrate buffer (pH 3.0) was added to the reaction liquid to stop the reaction. An equivalent amount of water was added thereto, and 0.5 times by volume of an aqueous solution of 0.1% 3-methyl-2-benzothiazolinon hydrazone hydrochloride monohydrate was added, absorbance at 305 nm was measured to determine a concentration of 3-hydroxypropionaldehyde. As a result, it was confirmed that 0.196 M of 3-hydroxypropionaldehyde was formed in the reaction mixture. A conversion ratio of glycerin to 3-hydroxypropionaldehyde was 98% . The residual reaction mixture was filtered to remove toluene-treated microbial cells. After the filtrate was placed into a test tube with a sealed cap, 0.015 g of 5% palladium carbon was added thereto, and a gas phase part was replaced with hydrogen gas. A balloon was filled with 500 ml of hydrogen gas under normal pressure, connected to the gas phase part and sealed, and the reaction was carried out with stirring at 60°C for 5 hours in a hot-water bath. Analysis of the reaction mixture indicated that 0.196 M of 1,3-propandiol was formed. In this case, catalyst amount of microbial cells [X (U/g glycerin)], concentration of glycerin [Y (g/100 ml)], and X/Y² are shown in Table 1 below.

### Example 3

In the same manner as in Example 2, 0.196 M of 3-hydroxypropionaldehyde (conversion ratio of glycerin: 98%) was produced. The reaction mixture containing the same was adjusted to pH 2 with 35% hydrochloric acid, and allowed to stand at room temperature for 1 hour. The acrolein formed in the reaction mixture was quantitatively determined, to find that 0.130 M of acrolein was formed.

### Example 4

In the same manner as in Example 1, 0.188 M of 3-hydroxypropionaldehyde(conversion ratio of glycerin:94%) was produced. After the reaction mixture was placed into a test tube with a sealed cap, 0.015 g of 5% palladium carbon was added thereto, and a gas phase part was replaced with oxygen gas. A balloon was filled with 1 litter of oxygen gas under normal pressure, connected to the gas phase part and sealed, and the reaction was carried out with stirring at 60°C for 5 hours. Analysis of the reaction mixture indicated that 0.150 M of 3-hydroxypropionic acid was formed.

### Example 5

In the same manner as in Example 2, 0.196 M of 3-hydroxypropionaldehyde (conversion ratio of glycerin: 98%) was produced. 10 ml of the reaction mixture containing the same was adjusted to pH 2 with 35% hydrochloric acid, and allowed to stand at room temperature for 1 hour. The acrolein formed in the reaction mixture was quantitatively determined, to find that 0.150 M of acrolein was formed.

Subsequently, methanol was added to the resultant acrolein. The reaction was performed with stirring thoroughly under an oxygen atmosphere using 0.015 g of 5% palladium carbon as an oxidation catalyst, to form 0.188 M of methyl acrylate.

### Example 6

In the same manner as in Example 2, 0.196 M of 3-hydroxypropionaldehyde (conversion ratio of glycerin: 98%) was produced. The reaction mixture containing the same was adjusted to pH 2 with 35% hydrochloric acid, and allowed to stand at room temperature for 1 hour. The acrolein formed in the reaction mixture was quantitatively determined, to find that 0.148 M of acrolein (conversion ratio to 3-hydroxypropionaldehyde: 96.9%) was formed.

Subsequently, after the reaction mixture containing the resultant acrolein was placed into a test tube with a sealed cap, 0.015 g of 5% palladium carbon was added thereto, and a gas phase part was replaced with oxygen gas. A balloon was filled with 1 litter of oxygen gas under normal pressure, connected to the gas phase part and sealed, and the reaction was carried out with stirring at 60°C for 5 hours. Analysis of the reaction mixture indicated that 0.120 M of acrylic acid was formed.

### Example 7

Klebsiella pneumoniae (ATCC 25955) was anaerobically cultured in a medium containing glycerin as a carbon source to grow until reaching a logarithmic growth phase. The microbial cell culture at this stage was treated with 1% toluene in the same manner as described in Example 2, and the treated microbial cells were collected. The collected microbial cells of 20 g (wet weight, enzymatic activity 4000 U) of Klebsiella pneumoniae (ATCC 25955) were added to 1 litter of 50 mM potassium phosphate buffer (pH 8) containing 135 µM coenzyme B12 and 0.2 M glycerin, and the resultant mixture was reacted at 37°C for 120 minutes. After the reaction mixture was filtered to remove off the toluene-treated microbial cells, an aliquot thereof was taken out and an amount of 3-hydroxypropionaldehyde therefor was quantitatively determined to find that 0.196 M of 3-hydroxypropionaldehyde was formed. The resultant 3-hydroxypropionaldehyde was placed into a 1 litter of separable flask, 1.5 g of 5% palladium carbonawas added thereto, and a gas phase part was replaced with oxygen. A small amount of oxygen was passed through the gas phase part in order to prevent the penetration of outside gas. The mixture was reacted at 60°C for 5 hours with stirring. The reaction mixture was analyzed to detect 0.178 M of 3-hydroxypropionic acid. Catalyst amount of the toluene-treated microbial cells [X (U/g glycerin)], concentration of glycerin [Y (g/100 ml)], and X/Y² are summarized in Table 1 below.

### Example 8

Klebsiella pneumoniae (ATCC 25955) was anaerobically cultured in a medium containing glycerin as a carbon source to grow until reaching a logarithmic growth phase. The microbial cell culture at this stage was treated with 1% toluene in the same manner as described in Example 2, and the treated microbial cells were collected. The collected microbial cells of 20 g (wet weight, enzymatic activity 4000 U) of Klebsiella pneumoniae (ATCC 25955) were added to 1 litter of 50 mM potassium phosphate buffer (pH 8) containing 135 µM coenzyme B12 and 0.2 M glycerin, and the resultant mixture was reacted at 37°C for 120 minutes. After the reaction mixture was filtered to remove off the toluene-treated microbial cells, an aliquot thereof was taken out and an amount of 3-hydroxypropionaldehyde therefor was quantitatively determined to find that 0.197 M of 3-hydroxypropionaldehyde was formed. The resultant reaction mixture containing 3-hydroxypropionaldehyde was adjusted to pH 2 with 35% hydrochloric acid, and allowed to stand at room temperature for 1 hour. An amount of acrolein formed in the reaction mixture was quantitatively determined, to find that 0.130 M of acrolein was formed. Catalyst amount of the toluene-treated microbial cells (X (U/g glycerin)), concentration of glycerin (Y (g/100 ml)), and X/Y² are summarized in Table 1 below.

### [Table 1]

**Table 1**

| Source | Catalytic amount X (U/g Gly) | Substrate conc. Y (%) | X/Y² | Conversion (%) |
|---|---|---|---|---|
| J. Bac. Vol.181, No.13, '99, p.4110- 4113 | 8 | 0.92 | 8.989 | 1.4 |
| The J. of Biolog. Chem., Vol.272, No.51, '97 | 1 | 1.84 | 0.273 | 0.17 |
| Arch. Microbiol., 174, 81-88 (2000) | 8 | 0.92 | 8.989 | 1.4 |
| The J. of Biolog. Chem., Vol.274, No.6, '99 | 1 | 11.04 | 0.010 | 0.27 |
| The J. of Biolog. Chem., Vol.274, No.6, '99 | 7 | 13.8 | 0.034 | 0.75 |
| Example 1 | 91 | 1.84 | 27 | 50 |
| Example 2 | 533 | 1.84 | 157 | 98 |
| Example 3 | 533 | 1.84 | 157 | 98 |
| Example 7 | 217 | 1.84 | 64 | 98 |
| Example 8 | 217 | 1.84 | 64 | 98.5 |

### Example 9

To 10 ml of 50 mM potassium phosphate buffer (pH 8) containing 4.6% (0.5 M) of glycerin as a substrate and 135 µM of coenzyme B12, an enzymatic activity 200 U/g wet weight of toluene-treated (Klebsiella pneumoniae) microbial cells were added so as to give a catalyst amount as shown in Table 2 below. The mixture was reacted with stirring at 37°C for 6 hours in dark. The toluene-treated microbial cells were removed off by centrifugation of the reaction mixture after performing the reaction for a prescribed time. Then, the conversion ratio from glycerin to 3-hydroxypropionaldehyde was determined using the supernatant. Catalyst amount of the toluene-treated microbial cells [X (U/g glycerin)], concentration of glycerin [Y (g/100 ml)], and X/Y² are summarized in Table 2 below.

### [Table 2]

**Table 2**

| Amount of microbial cells (g) | Catalytic amount X (U/g glycerin) | Substrate conc. Y (%) | X/Y², | Conversion (%) |
|---|---|---|---|---|
| 0.1 | 920 | 4.6 | 43 | 52 |
| 0.2 | 1840 | 4.6 | 87 | 89 |
| 0.3 | 1304 | 4.6 | 95 | 96 |
| 0.5 | 2174 | 4.6 | 103 | 98 |
| 1 | 4348 | 4.6 | 205 | 98 |
| 2 | 8696 | 4.6 | 411 | 98 |
| 5 | 21739 | 4.6 | 1027 | 97 |
| 10 | 43478 | 4.6 | 2055 | 98 |
| 15 | 65217 | 4.6 | 3082 | 95 |
| 20 | 86957 | 4.6 | 4109 | 95 |
| 25 | 108696 | 4.6 | 5137 | 90 |
| 30 | 130435 | 4.6 | 6164 | 85 |
| 35 | 152174 | 4.6 | 7192 | 70 |
| 40 | 173913 | 4.6 | 8219 | 65 |
| 45 | 195652 | 4.6 | 9146 | 51 |
| 50 | 217391 | 4.6 | 10274 | 30 |

As shown in the results of the Table 2, when X/Y² exceeds 80, a high conversion ratio as of about not less than 90% can be achieved. When the value is not more than 8219, it is shown that the conversion ratio is also below 70%. Further, when X/Y² is within a range of 87 to 6164, a conversion ratio of not less than 80% can be achieved, especially when X/Y² is in a range of 95 to 5137, it is shown that an extremely high conversion ratio not less than 80% can be achieved.

### Example 10

A toluene-treated microbial cell culture of a strain JM 109/vector 1' (GD) was prepared by the same way as described in Example 2. The toluene-treated microbialcells(enzymatic activity 9, 900 U/g wet weight) was added into 50 mM potassium phosphate buffer (pH 8) containing 9.2% (1 M) of glycerin as a substrate and 135 µM of coenzyme B12 so as to give a catalytic amount as shown in Table 3 below, and the total volume was adjusted to 10 ml. The mixture was reacted at 37°C for 2 hours in dark. The toluene-treated microbial cells were removed off by centrifugation of the reaction mixture after performing the reaction for a prescribed time, and a conversion ratio from glycerin to 3-hydroxypropionaldehyde was determined using the supernatant. Catalyst amount of the toluene-treated microbial cells [X (U/g glycerin)], concentration of glycerin [Y (g/100 ml)], and X/Y² are summarized in Table 3 below.

### [Table 3]

**Table 3**

| Amount of microbial cells (g) | Catalytic amount X (U/g glycerin) | Substrate conc. (Y) (%) | X/Y² | Conversion (%) |
|---|---|---|---|---|
| 0.051 | 543 | 9.2 | 6 | 43.8 |
| 0.101 | 1087 | 9.2 | 13 | 72.1 |
| 0.202 | 2174 | 9.2 | 26 | 79.3 |
| 0.303 | 3261 | 9.2 | 39 | 79.4 |
| 0.404 | 4348 | 9.2 | 51 | 83.3 |
| 0.752 | 8096 | 9.2 | 77 | 78.3 |

As shown in the result of the Table 3, when X/Y² exceeds 10, a high conversion ratio as of not less than 70% can be achieved. Especially when X/Y² exceeds 50, it is shown that an extremely high Conversion ratio as of not less than 80% can be achieved. Further, it is noted in this Example that even if a considerably high concentration of substrate as high as 9.2% is used, an conversion ratio of not less than 70% can be achieved by applying an action of the toluene-treated microbial cells to glycerin under conditions that X/Y² becomes not less than 10. In addition, as shown in this Example, according to the method of the present invention, since a high conversion ratio can be achieved even in a high concentration of substrate, it is suggested that the method of the present invention is very advantageous from the viewpoint of an industrial level.

### Industrial Applicability

According to the method for producing 3-hydroxypropionaldehyde of the present invention, by acting diol/glycerol dehydratase and/or diol/glycerol dehydratase reactivating factor on 3-HPA while an amount of enzyme being controlled within a specified range, the reaction from glycerin to 3-HPA occurs selectively without inducing any side reaction other than the reaction from glycerin to 3-HPA, such a high conversion ratio as not less than 80%, in some cases, not less than 90% can be attained, and 3-hydroxypropionaldehyde can be produced in a high yield. Further, in the method of the present invention, since the reaction from glycerin to 3-HPA can be performed without using a fermentation method, 3-hydroxypropionaldehyde can be produced with a high purity scarcely containing by-product. Accordingly, 3-HPA obtained by the method of the present invention can be used as an intermediate in producing 1,3-propanediol by hydrogenation, through a very simple purification process for separation of microbial cell / treated microbial cell such as filtration, ultrafiltration and settling, and the like. In addition to the advantage, since only 1,3-propanediol and water mainly remain after completion of the reaction, and no organic solvents are needed for purification, the method does not require recovery of organic solvent and post-treatment, which is preferable from the viewpoint of environment.

Further, the present invention relates to a method for obtaining acrolein by reacting the 3-hydroxypropionaldehyde scarcely containing by-product as described above under acidic conditions, and further for producing acrylic acid by oxidizing the acrolein; and a method for producing an acrylic ester by reacting 3-hydroxypropionaldehyde scarcely containing by-product as described above under acidic conditions to obtain acrolein, and further subjecting the acrolein to oxidative esterification. According to the method, since 3-hydroxypropionaldehyde as a raw material scarcely contains by-product, acrolein produced using this, and further acrylic acid and an acrylic ester produced from this have also high purities.

According to the present invention, since 3-HPA can be produced in a high conversion ratio and a high purity ratio, and scarcely containing by-product, 1,3-propanediol, 3-hydroxypropionic acid, acrolein, acrylic acid and acrylic ester can be produced in high yields and with high purities, respectively. Accordingly, 1,3-propanediol thus produced can be used as a monomer to be used for producing polyesters and polyurethanes, and as a starting material for synthesis of cyclic compounds. Further, fibers produced by using this compound do not show discoloration because 1,3-propanediol scarcely contains by-product. Also, acrylic acid / acrylic ester thus produced can be used not only for copolymers for acrylic fibers or for adhesives / agglutinant as an emulsion, but also for coating materials, textile processing, leathers, construction materials, and the like.

### Brief Description of the Drawings

[Fig. 1A]
   Fig. 1A shows a gene map of vector 1, which is an expressing plasmid used in Example 1. In Fig. 1A, Amp^{r} represents an ampicillin-resistant gene; Cm^{r} represents a chloramphenicol-resistant gene; lacIq represents a lactose repressor gene; and P_{tac} represents a tac promoter, respectively.
[Fig. 1B]
   Fig. 1B shows a gene map of vector 2, which is an expressing plasmid used in Example 1. In Fig. 1B, Amp^{r} represents an ampicillin-resistant gene; Cm^{r} represents a chloramphenicol-resistant gene; lacI_{q} represents a lactose repressor gene; and P_{tac} represents a tac promoter, respectively.
[Fig. 2A]
   Fig. 1B shows a gene map of vector 1' , which is an expressing plasmid used in Example 2. In Fig. 2A, Amp^{r} represents an ampicillin-resistant gene; Cm^{r} represents a chloramphenicol-resistant gene; lacIq represents a lactose repressor gene; and P_{tac} represents a tac promoter, respectively.
[Fig. 2B]
   Fig. 1B shows a gene map of vector 2' , which is an expressing plasmid used in Example 2. In Fig. 2A, Amp^{r} represents an ampicillin-resistant gene; Cm^{r} represents a chloramphenicol-resistant gene; laciq represents a lactose repressor gene; and P_{tac} represents a tac promoter, respectively.

## Claims

**1.** A method for producing 3-hydroxypropionaldehyde which comprises a step of dehydrating glycerin using a microbial cell and/or a treated microbial cell containing diol dehydratase and/or glycerol dehydratase, and optionally diol dehydratase reactivating factor and/or glycerol dehydratase reactivating factor, under conditions so as to give a value (X/Y²) calculated by dividing a catalytic amount [X (U/g glycerin)] of diol dehydratase and/or glycerol dehydratase by square of glycerin concentration [Y (g/100 ml)] within a range of 10 to 8, 000, to produce 3-hydroxypropionaldehyde.

**2.** A method according to claim 1, wherein the dehydration of glycerin is performed using a microbial cell under aerobic conditions.

**3.** A method according to claim 1, wherein the dehydration of glycerin is performed using a treated microbial cell.

**4.** A method for producing 1, 3-propanediol which comprises a step of removing the microbial cell and/or treated microbial cell from the 3-hydroxypropionaldehyde produced by the method set forth in any one of claims 1 to 3, subsequently hydrogenating said 3-hydroxypropionaldehyde to produce 1,3-propanediol.

**5.** A method for producing 3-hydroxypropionic acid which comprises a step of oxidizing the 3-hydroxypropionaldehyde produced by the method set forth in any one of claims 1 to 3 to produce 3-hydroxypropionic acid.

**6.** A method for producing acrolein which comprises a step of reacting the 3-hydroxypropionaldehyde produced by the method set forth in any one of claims 1 to 3 under acidic conditions, to produce acrolein.

**7.** A method for producing acrylic acid which comprises a step of oxidizing the acrolein produced by the method set forth in claim 6 to produce acrylic acid.

**8.** A method for producing an acrylic ester which comprises a step of subjecting the acrolein produced by the method set forth in claim 6 to the oxidative esterification, to produce an acrylic ester.


SEQUENCE LISTING

<110> Nippon Shokubai Co., Ltd.

<120> Method for Production of 3-hydroxypropionaldehyde

<130> F04-047

<150> JP 2003-337663
<151> 2003-09-29

<160> 4

<170> PatentIn version 3.1

<210> 1
<211> 7183
<212> DNA
<213> plasmid pBR322

<400> 1
acgttatcga ctgcacggtg caccaatgct tctggcgtca ggcagccatc ggaagctgtg 60

gtatggctgt gcaggtcgta aatcactgca taattcgtgt cgctcaaggc gcactcccgt 120

tctggataat gttttttgcg ccgacatcat aacggttctg gcaaatattc tgaaatgagc 180

tgttgacaat taatcatcgg ctcgtataat gtgtggaatt gtgagcggat aacaatttca 240

cacaggaaac agtacatatg agatcgaaaa gatttgaagc actggcgaaa cgccctgtga 300

atcaggacgg cttcgttaag gagtggatcg aagaaggctt tatcgcgatg gaaagcccga 360

acgacccaaa accgtcgatt aaaatcgtta acggcgcggt gaccgagctg gacgggaaac 420

cggtaagcga ttttgacctg atcgaccact ttatcgcccg ctacggtatc aacctgaacc 480

gcgccgaaga agtgatggcg atggattcgg tcaagctggc caacatgctg tgcgatccga 540

acgttaaacg cagcgaaatc gtcccgctga ccaccgcgat gacgccggcg aaaattgtcg 600

aagtggtttc gcatatgaac gtcgtcgaga tgatgatggc gatgcagaaa atgcgcgccc 660

gccgcacccc gtcccagcag gcgcacgtca ccaacgtcaa agataacccg gtacagattg 720

ccgccgacgc cgccgaaggg gcatggcgcg gatttgacga acaggaaacc accgttgcgg 780

tagcgcgcta tgcgccgttc aacgccatcg cgctgctggt gggctcgcag gtaggccgtc 840

cgggcgtgct gacgcagtgc tcgctggaag aagccaccga gctgaagctc ggcatgctgg 900

gccacacctg ctacgccgaa accatctccg tctacggcac cgagccggtc tttaccgacg 960

gcgacgacac gccgtggtcg aagggcttcc tcgcctcgtc ctacgcctct cgcgggctga 1020

aaatgcgctt tacctccggc tccggctcgg aagtgcagat gggctacgcc gaaggcaaat 1080

ccatgcttta tctggaagcg cgctgcatct acatcaccaa agccgcgggc gtacagggtc 1140

tgcaaaacgg ttccgtaagc tgcatcggcg tgccgtctgc ggtgccttcc ggcattcgcg 1200

cggtgctggc ggaaaacctg atctgttcgt cgctggatct ggagtgcgcc tccagcaacg 1260

accagacctt cacccactcc gatatgcgtc gtaccgcgcg cctgctgatg cagttcctgc 1320

cgggcaccga ctttatctcc tccggttatt ccgcggtgcc gaactacgac aacatgttcg 1380

ccggctccaa cgaagatgcc gaagactttg acgactacaa cgtcatccag cgcgacctga 1440

aggtggacgg cggtttgcgt ccggttcgcg aagaggacgt catcgccatc cgtaacaaag 1500
ccgcccgcgc gctgcaggcc gtgtttgccg gaatggggct gccgccgatt accgatgaag 1560
aagttgaagc cgcgacctac gcccacggtt cgaaagatat gccggagcgc aacatcgtcg 1620
aagacatcaa gttcgcccag gaaatcatca ataaaaaccg caacggtctg gaagtggtga 1680
aagcgctggc gcagggcgga ttcaccgacg tggcccagga catgctcaac atccagaaag 1740
ctaagctgac cggggactac ctgcatacct ccgcgattat cgtcggcgac gggcaggtgc 1800
tgtcagccgt caacgacgtc aacgactatg ccggtccggc aacgggctat cgcctgcagg 1860
gcgaacgctg ggaagagatt aaaaacatcc ctggcgctct tgatcccaac gagattgatt 1920
aaggggtgag aaatggaaat taatgaaaaa ttgctgcgcc agataattga agacgtgctc 1980
agcgagatga agggcagcga taaaccggtc tcgtttaatg cgccggcggc ctccgcggcg 2040
ccccaggcca cgccgcccgc cggcgacggc ttcctgacgg aagtgggcga agcgcgtcag 2100
ggaacccagc aggacgaagt gattatcgcc gtcggcccgg ctttcggcct ggcgcagacc 2160
gtcaatatcg tcggcatccc gcataagagc attttgcgcg aagtcattgc cggtattgaa 2220
gaagaaggca ttaaggcgcg cgtgattcgc tgctttaaat cctccgacgt ggccttcgtc 2280
gccgttgaag gtaatcgcct gagcggctcc ggcatctcta tcggcatcca gtcgaaaggc 2340
accacggtga tccaccagca ggggctgccg ccgctctcta acctggagct gttcccgcag 2400
gcgccgctgc tgaccctgga aacctatcgc cagatcggca aaaacgccgc ccgctatgcg 2460
aaacgcgaat cgccgcagcc ggtcccgacg ctgaatgacc agatggcgcg gccgaagtac 2520
caggcgaaat cggccatttt gcacattaaa gagaccaagt acgtggtgac gggcaaaaac 2580
ccgcaggaac tgcgcgtggc gctttgataa aggataactc catgaatacc gacgcaattg 2640
aatcgatggt acgcgacgta ttgagccgca tgaacagcct gcagggcgag gcgcctgcgg 2700
cggctccggc ggctggcggc gcgtcccgta gcgccagggt cagcgactac ccgctggcga 2760
acaagcaccc ggaatgggtg aaaaccgcca ccaataaaac gctggacgac tttacgctgg 2820
aaaacgtgct gagcaataaa gtcaccgccc aggatatgcg tattaccccg gaaaccctgc 2880
gcttacaggc ttctattgcc aaagacgcgg gccgcgaccg gctggcgatg aacttcgagc 2940
gcgccgccga gctgaccgcg gtaccggacg atcgcattct tgaaatctac aacgccctcc 3000
gcccctatcg ctcgacgaaa gaggagctgc tggcgatcgc cgacgatctc gaaagccgct 3060
atcaggcgaa gatttgcgcc gctttcgttc gcgaagcggc cacgctgtac gtcgagcgta 3120
aaaaactcaa aggcgacgat taacttcatt ccgggcccgt cgacagatcc ccgggaattc 3180
atcgtgactg actgacgatc tgcctcgcgc gtttcggtga tgacggtgaa aacctctgac 3240
acatgcagct cccggagacg gtcacagctt gtctgtaagc ggatgccggg agcagacaag 3300
cccgtcaggg cgcgtcagcg ggtgttggcg ggtgtcgggg cgcagccatg acccagtcac 3360
gtagcgatag cggagtgtat aattcttgaa gacgaaaggg cctcgtgata cgcctatttt 3420
tataggttaa tgtcatgata ataatggttt cttagacgtc aggtggcact tttcggggaa 3480
atgtgcgcgg aacccctatt tgtttatttt tctaaataca ttcaaatatg tatccgctca 3540

tgagacaata accctgataa atgcttcaat aatattgaaa aaggaagagt atgagtattc 3600
aacatttccg tgtcgccctt attccctttt ttgcggcatt ttgccttcct gtttttgctc 3660
acccagaaac gctggtgaaa gtaaaagatg ctgaagatca gttgggtgca cgagtgggtt 3720
acatcgaact ggatctcaac agcggtaaga tccttgagag ttttcgcccc gaagaacgtt 3780
ttccaatgat gagcactttt aaagttctgc tatgtggcgc ggtattatcc cgtgttgacg 3840
ccgggcaaga gcaactcggt cgccgcatac actattctca gaatgacttg gttgagtact 3900
caccagtcac agaaaagcat cttacggatg gcatgacagt aagagaatta tgcagtgctg 3960
ccataaccat gagtgataac actgcggcca acttacttct gacaacgatc ggaggaccga 4020
aggagctaac cgcttttttg cacaacatgg gggatcatgt aactcgcctt gatcgttggg 4080
aaccggagct gaatgaagcc ataccaaacg acgagcgtga caccacgatg cctgcagcaa 4140
tggcaacaac gttgcgcaaa ctattaactg gcgaactact tactctagct tcccggcaac 4200
aattaataga ctggatggag gcggataaag ttgcaggacc acttctgcgc tcggcccttc 4260
cggctggctg gtttattgct gataaatctg gagccggtga gcgtgggtct cgcggtatca 4320
ttgcagcact ggggccagat ggtaagccct cccgtatcgt agttatctac acgacgggga 4380
gtcaggcaac tatggatgaa cgaaatagac agatcgctga gataggtgcc tcactgatta 4440
agcattggta actgtcagac caagtttact catatatact ttagattgat ttaaaacttc 4500
atttttaatt taaaaggatc taggtgaaga tcctttttga taatctcatg accaaaatcc 4560
cttaacgtga gttttcgttc cactgagcgt cagaccccgt agaaaagatc aaaggatctt 4620
cttgagatcc tttttttctg cgcgtaatct gctgcttgca aacaaaaaaa ccaccgctac 4680
cagcggtggt ttgtttgccg gatcaagagc taccaactct ttttccgaag gtaactggct 4740
tcagcagagc gcagatacca aatactgtcc ttctagtgta gccgtagtta ggccaccact 4800
tcaagaactc tgtagcaccg cctacatacc tcgctctgct aatcctgtta ccagtggctg 4860
ctgccagtgg cgataagtcg tgtcttaccg ggttggactc aagacgatag ttaccggata 4920
aggcgcagcg gtcgggctga acggggggtt cgtgcacaca gcccagcttg gagcgaacga 4980
cctacaccga actgagatac ctacagcgtg agctatgaga aagcgccacg cttcccgaag 5040
ggagaaaggc ggacaggtat ccggtaagcg gcagggtcgg aacaggagag cgcacgaggg 5100
agcttccagg gggaaacgcc tggtatcttt atagtcctgt cgggtttcgc cacctctgac 5160
ttgagcgtcg atttttgtga tgctcgtcag gggggcggag cctatggaaa aacgccagca 5220
acgcggcctt tttacggttc ctggcctttt gctggccttt tgctcacatg ttctttcctg 5280
cgttatcccc tgattctgtg gataaccgta ttaccgcctt tgagtgagct gataccgctc 5340
gccgcagccg aacgaccgag cgcagcgagt cagtgagcga ggaagcggaa gagcgcctga 5400
tgcggtattt tctccttacg catctgtgcg gtatttcaca ccgcataaat tccgacacca 5460
tcgaatggtg caaaaccttt cgcggtatgg catgatagcg cccggaagag agtcaattca 5520
gggtggtgaa tgtgaaacca gtaacgttat acgatgtcgc agagtatgcc ggtgtctctt 5580

atcagaccgt ttcccgcgtg gtgaaccagg ccagccacgt ttctgcgaaa acgcgggaaa 5640
aagtggaagc ggcgatggcg gagctgaatt acattcccaa ccgcgtggca caacaactgg 5700
cgggcaaaca gtcgttgctg attggcgttg ccacctccag tctggccctg cacgcgccgt 5760
cgcaaattgt cgcggcgatt aaatctcgcg ccgatcaact gggtgccagc gtggtggtgt 5820
cgatggtaga acgaagcggc gtcgaagcct gtaaagcggc ggtgcacaat cttctcgcgc 5880
aacgcgtcag tgggctgatc attaactatc cgctggatga ccaggatgcc attgctgtgg 5940
aagctgcctg cactaatgtt ccggcgttat ttcttgatgt ctctgaccag acacccatca 6000
acagtattat tttctcccat gaagacggta cgcgactggg cgtggagcat ctggtcgcat 6060
tgggtcacca gcaaatcgcg ctgttagcgg gcccattaag ttctgtctcg gcgcgtctgc 6120
gtctggctgg ctggcataaa tatctcactc gcaatcaaat tcagccgata gcggaacggg 6180
aaggcgactg gagtgccatg tccggttttc aacaaaccat gcaaatgctg aatgagggca 6240
tcgttcccac tgcgatgctg gttgccaacg atcagatggc gctgggcgca atgcgcgcca 6300
ttaccgagtc cgggctgcgc gttggtgcgg atatctcggt agtgggatac gacgataccg 6360
aagacagctc atgttatatc ccgccgttaa ccaccatcaa acaggatttt cgcctgctgg 6420
ggcaaaccag cgtggaccgc ttgctgcaac tctctcaggg ccaggcggtg aagggcaatc 6480
agctgttgcc cgtctcactg gtgaaaagaa aaaccaccct ggcgcccaat acgcaaaccg 6540
cctctccccg cgcgttggcc gattcattaa tgcagctggc acgacaggtt tcccgactgg 6600
aaagcgggca gtgagcgcaa cgcaattaat gtgagttagc tcactcatta ggcaccccag 6660
gctttacact ttatgcttcc ggctcgtatg ttgtgtggaa ttgtgagcgg ataacaattt 6720
cacacaggaa acagctatga ccatgattac ggattcactg gccgtcgttt tacaacgtcg 6780
tgactgggaa aaccctggcg ttacccaact taatcgcctt gcagcacatc cccctttcgc 6840
cagctggcgt aatagcgaag aggcccgcac cgatcgccct tcccaacagt tgcgcagcct 6900
gaatggcgaa tggcgctttg cctggtttcc ggcaccagaa gcggtgccgg aaagctggct 6960
ggagtgcgat cttcctgagg ccgatactgt cgtcgtcccc tcaaactggc agatgcacgg 7020
ttacgatgcg cccatctaca ccaacgtaac ctatcccatt acggtcaatc cgccgtttgt 7080
tcccacggag aatccgacgg gttgttactc gctcacattt aatgttgatg aaagctggct 7140
acaggaaggc cagacgcgaa ttatttttga tggcgttgga att 7183

<210> 2
<211> 6607
<212> DNA
<213> plasmid p15A

<400> 2
gaattaattc tggcgaatcc tctgaccagc cagaaaacga cctttctgtg gtgaaaccgg 60
atgctgcaat tcagagcgcc agcaagtggg ggacagcaga agacctgacc gccgcagagt 120
ggatgtttga catggtgaag actatcgcac catcagccag aaaaccgaat tttgctgggt 180
gggctaacga tatccgcctg atgcgtgaac gtgacggacg taaccaccgc gacatgtgtg 240

tgctgttccg ctgggcatgc caggacaact tctggtccgg taacgtgctg agcccggcca 300
agcttactcc ccatccccct gttgacaatt aatcatcggc tcgtataatg tgtggaattg 360
tgagcggata acaatttcac acaggaaaca ggatcctagg aggtttaaac atatgcgata 420
tatagctggc attgatatcg gcaactcatc gacggaagtc gccctggcga ccctggatga 480
ggctggcgcg ctgacgatca cccacagcgc gctggcggaa accaccggaa tcaaaggcac 540
gttgcgtaac gtgttcggga ttcaggaggc gctcgccctc gtcgccagag gcgccgggat 600
cgccgtcagc gatatttcgc tcatccgcat caacgaagcg acgccggtga ttggcgatgt 660
ggcgatggaa accattaccg aaaccatcat caccgaatcg accatgatcg gccataaccc 720
gaaaacgccc ggcggcgcgg ggcttggcac aggcatcacc attacgccgc aggagctgct 780
aacccgcccg gcggacgcgc cctatatcct ggtggtgtcg tcggcgttcg attttgccga 840
tatcgccagc gtgattaacg cttccctgcg cgccgggtat cagattaccg gcgtcatttt 900
acagcgcgac gatggcgtgc tggtcagcaa ccggctggaa aaaccgctgc cgatcgttga 960
cgaagtgctg tacatcgacc gcattccgct ggggatgctg gcggcgattg aggtcgccgt 1020
tccggggaag gtcatcgaaa ccctctctaa cccttacggc atcgccaccg tctttaacct 1080
cagccccgag gagacgaaga acatcgtccc gatggcccgg gcgctgattg gcaaccgttc 1140
cgccgtggtg gtcaaaacgc catccggcga cgtcaaagcg cgcgcgatac ccgccggtaa 1200
tcttgagctg ctggcccagg gccgtagcgt gcgcgtggat gtggccgccg gcgccgaagc 1260
catcatgaaa gcggtcgacg gctgcggcag gctcgataac gtcaccggcg aatccggcac 1320
caatatcggc ggcatgctgg aacacgtgcg ccagaccatg gccgagctga ccaacaagcc 1380
gagcagcgaa atatttattc aggacctgct ggccgttgat acctcggtac cggtgagcgt 1440
taccggcggt ctggccgggg agttctcgct ggagcaggcc gtgggcatcg cctcgatggt 1500
gaaatcggat cgcctgcaga tggcaatgat cgcccgcgaa atcgagcaga agctcaatat 1560
cgacgtgcag atcggcggcg cagaggccga agccgccatc ctgggggcgc tgaccacgcc 1620
gggcaccacc cgaccgctgg cgatcctcga cctcggcgcg ggctccaccg atgcctccat 1680
catcaacccc aaaggcgaca tcatcgccac ccatctcgcc ggcgcaggcg acatggtgac 1740
gatgattatt gcccgcgagc tggggctgga agaccgctat ctggcggaag agatcaagaa 1800
gtacccgctg gctaaggtgg aaagcctgtt ccatttacgc cacgaggacg gcagcgtgca 1860
gttcttctcc acgccgctgc cgcccgccgt gttcgcccgc gtctgcgtgg tgaaagcgga 1920
cgaactggtg ccgctgcccg gcgatttagc gctggaaaaa gtgcgcgcca ttcgccgcag 1980
cgccaaagag cgggtctttg tcaccaacgc cctgcgcgcg ctgcgtcagg tcagccccac 2040
cggcaacatt cgcgatattc cgttcgtggt gctggtcggc ggttcgtcgc tggatttcga 2100
agtcccgcag ctggtcaccg atgcgctggc gcactaccgc ctggttgccg gacggggaaa 2160
tattcgcggc agcgagggcc cccgaaacgc ggtggccacc ggcctgattc tctcctggca 2220
taaggagttt gcgcatgaac ggtaatcaca gcgccccggc catcgcgatc gccgtcatcg 2280
acggctgcga cggcctgtgg cgcgaagtgc tgctgggtat cgaagaggaa ggtatccctt 2340

tccggctcca gcatcacccg gccggagagg tcgtggacag cgcctggcag gcggcgcgca 2400
gctcgccgct gctggtgggc atcgcctgcg accgccatat gctggtcgtg cactacaaga 2460
atttacccgc atcggcgccg ctttttacgc tgatgcatca tcaggacagt caggcccatc 2520
gcaacaccgg taataacgcg gcacggctgg tcaaggggat ccctttccgg gatctgaata 2580
gcgaagcaac aggagaacag caggatgaat aagatctcgg gtagcccgcc taatgagcgg 2640
gctttttttt atgagaatta caacttatat cgtatggggc tgacttcagg tgctacattt 2700
gaagagataa attgcactga aatctagaaa tattttatct gattaataag atgatcttct 2760
tgagatcgtt ttggtctgcg cgtaatctct tgctctgaaa acgaaaaaac cgccttgcag 2820
ggcggttttt cgaaggttct ctgagctacc aactctttga accgaggtaa ctggcttgga 2880
ggagcgcagt caccaaaact tgtcctttca gtttagcctt aaccggcgca tgacttcaag 2940
actaactcct ctaaatcaat taccagtggc tgctgccagt ggtgcttttg catgtctttc 3000
cgggttggac tcaagacgat agttaccgga taaggcgcag cggtcggact gaacgggggg 3060
ttcgtgcata cagtccagct tggagcgaac tgcctacccg gaactgagtg tcaggcgtgg 3120
aatgagacaa acgcggccat aacagcggaa tgacaccggt aaaccgaaag gcaggaacag 3180
gagagcgcac gagggagccg ccagggggaa acgcctggta tctttatagt cctgtcgggt 3240
ttcgccacca ctgatttgag cgtcagattt cgtgatgctt gtcagggggg cggagcctat 3300
ggaaaaacgg ctttgccgcg gccctctcac ttccctgtta agtatcttcc tggcatcttc 3360
caggaaatct ccgccccgtt cgtaagccat ttccgctcgc cgcagtcgaa cgaccgagcg 3420
tagcgagtca gtgagcgagg aagcggaata tatcctgtat cacatattct gctgacgcac 3480
cggtgcagcc ttttttctcc tgccacatga agcacttcac tgacaccctc atcagtgcca 3540
acatagtaag ccagtataca ctccgctagc gctgatgtcc ggcggtgctt ttgccgttac 3600
gcaccacccc gtcagtagct gaacaggagg gacagctgat agaaacagaa gccactggag 3660
cacctcaaaa acaccatcat acactaaatc agtaagttgg cagcatcacc cgacgcactt 3720
tgcgccgaat aaatacctgt gacggaagat cacttcgcag aataaataaa tcctggtgtc 3780
cctgttgata ccgggaagcc ctgggccaac ttttggcgaa aatgagacgt tgatcggcac 3840
gtaagaggtt ccaactttca ccataatgaa ataagatcac taccgggcgt attttttgag 3900
ttatcgagat tttcaggagc taaggaagct aaaatggaga aaaaaatcac tggatatacc 3960
accgttgata tatcccaatg gcatcgtaaa gaacattttg aggcatttca gtcagttgct 4020
caatgtacct ataaccagac cgttcagctg gatattacgg cctttttaaa gaccgtaaag 4080
aaaaataagc acaagtttta tccggccttt attcacattc ttgcccgcct gatgaatgct 4140
catccggaat ttcgtatggc aatgaaagac ggtgagctgg tgatatggga tagtgttcac 4200
ccttgttaca ccgttttcca tgagcaaact gaaacgtttt catcgctctg gagtgaatac 4260
cacgacgatt tccggcagtt tctacacata tattcgcaag atgtggcgtg ttacggtgaa 4320
aacctggcct atttccctaa agggtttatt gagaatatgt ttttcgtctc agccaatccc 4380

tgggtgagtt tcaccagttt tgatttaaac gtggccaata tggacaactt cttcgccccc 4440
gttttcacca tgggcaaata ttatacgcaa ggcgacaagg tgctgatgcc gctggcgatt 4500
caggttcatc atgccgtctg tgatggcttc catgtcggca gaatgcttaa tgaattacaa 4560
cagtactgcg atgagtggca gggcggggcg taattttttt aaggcagtta ttggtgccct 4620
taaacgcctg gtgctacgcc tgaataagtg ataataagcg gatgaatggc agaaattcga 4680
aagcaaattc gacccggtcg tcggttcagg gcagggtcgt taaatagccg cttatgtcta 4740
ttgctggttt accggtttat tgactaccgg aagcagtgtg accgtgtgct tctcaaatgc 4800
ctgaggccag tttgctcagg ctctccccgt ggaggtaata attgacgata tgatcattta 4860
ttctgcctcc caaagcaatt ccgacaccat cgaatggtgc aaaacctttc gcggtatggc 4920
atgatagcgc ccggaagaga gtcaattcag ggtggtgaat gtgaaaccag taacgttata 4980
cgatgtcgca gagtatgccg gtgtctctta tcagaccgtt tcccgcgtgg tgaaccaggc 5040
cagccacgtt tctgcgaaaa cgcgggaaaa agtggaagcg gcgatggcgg agctgaatta 5100
cattcccaac cgcgtggcac aacaactggc gggcaaacag tcgttgctga ttggcgttgc 5160
cacctccagt ctggccctgc acgcgccgtc gcaaattgtc gcggcgatta aatctcgcgc 5220
cgatcaactg ggtgccagcg tggtggtgtc gatggtagaa cgaagcggcg tcgaagcctg 5280
taaagcggcg gtgcacaatc ttctcgcgca acgcgtcagt gggctgatca ttaactatcc 5340
gctggatgac caggatgcca ttgctgtgga agctgcctgc actaatgttc cggcgttatt 5400
tcttgatgtc tctgaccaga cacccatcaa cagtattatt ttctcccatg aagacggtac 5460
gcgactgggc gtggagcatc tggtcgcatt gggtcaccag caaatcgcgc tgttagcggg 5520
cccattaagt tctgtctcgg cgcgtctgcg tctggctggc tggcataaat atctcactcg 5580
caatcaaatt cagccgatag cggaacggga aggcgactgg agtgccatgt ccggttttca 5640
acaaaccatg caaatgctga atgagggcat cgttcccact gcgatgctgg ttgccaacga 5700
tcagatggcg ctgggcgcaa tgcgcgccat taccgagtcc gggctgcgcg ttggtgcgga 5760
tatctcggta gtgggatacg acgataccga agacagctca tgttatatcc cgccgttaac 5820
caccatcaaa caggattttc gcctgctggg gcaaaccagc gtggaccgct tgctgcaact 5880
ctctcagggc caggcggtga agggcaatca gctgttgccc gtctcactgg tgaaaagaaa 5940
aaccaccctg gcgcccaata cgcaaaccgc ctctccccgc gcgttggccg attcattaat 6000
gcagctggca cgacaggttt cccgactgga aagcgggcag tgagcgcaac gcaattaatg 6060
tgagttagct cactcattag gcaccccagg ctttacactt tatgcttccg gctcgtatgt 6120
tgtgtggaat tgtgagcgga taacaatttc acacaggaaa cagctatgac catgattacg 6180
gattcactgg ccgtcgtttt acaacgtcgt gactgggaaa accctggcgt tacccaactt 6240
aatcgccttg cagcacatcc ccctttcgcc agctggcgta atagcgaaga ggcccgcacc 6300
gatcgccctt cccaacagtt gcgcagcctg aatggcgaat ggcgctttgc ctggtttccg 6360
gcaccagaag cggtgccgga aagctggctg gagtgcgatc ttcctgaggc cgatactgtc 6420
gtcgtcccct caaactggca gatgcacggt tacgatgcgc ccatctacac caacgtaacc 6480

tatcccatta cggtcaatcc gccgtttgtt cccacggaga atccgacggg ttgttactcg 6540

ctcacattta atgttgatga aagctggcta caggaaggcc agacgcgaat tatttttgat 6600

ggcgttg 6607

<210> 3
<211> 7023
<212> DNA
<213> plasmid pBR322

<400> 3
acgttatcga ctgcacggtg caccaatgct tctggcgtca ggcagccatc ggaagctgtg 60

gtatggctgt gcaggtcgta aatcactgca taattcgtgt cgctcaaggc gcactcccgt 120

tctggataat gttttttgcg ccgacatcat aacggttctg gcaaatattc tgaaatgagc 180

tgttgacaat taatcatcgg ctcgtataat gtgtggaatt gtgagcggat aacaatttca 240

cacaggaaac agtacatatg aaaagatcaa aacgatttgc agtactggcc cagcgccccg 300

tcaatcagga cgggctgatt ggcgagtggc ctgaagaggg gctgatcgcc atggacagcc 360

cctttgaccc ggtctcttca gtaaaagtgg acaacggtct gatcgtcgaa ctggacggca 420

aacgccggga ccagtttgac atgatcgacc gatttatcgc cgattacgcg atcaacgttg 480

agcgcacaga gcaggcaatg cgcctggagg cggtggaaat agcccgtatg ctggtggata 540

ttcacgtcag ccgggaggag atcattgcca tcactaccgc catcacgccg gccaaagcgg 600

tcgaggtgat ggcgcagatg aacgtggtgg agatgatgat ggcgctgcag aagatgcgtg 660

cccgccggac cccctccaac cagtgccacg tcaccaatct caaagataat ccggtgcaga 720

ttgccgctga cgccgccgag gccgggatcc gcggcttctc agaacaggag accacggtcg 780

gtatcgcgcg ctacgcgccg tttaacgccc tggcgctgtt ggtcggttcg cagtgcggcc 840

gccccggcgt gttgacgcag tgctcggtgg aagaggccac cgagctggag ctgggcatgc 900

gtggcttaac cagctacgcc gagacggtgt cggtctacgg caccgaagcg gtatttaccg 960

acggcgatga tacgccgtgg tcaaaggcgt tcctcgcctc ggcctacgcc tcccgcgggt 1020

tgaaaatgcg ctacacctcc ggcaccggat ccgaagcgct gatgggctat tcggagagca 1080

agtcgatgct ctacctcgaa tcgcgctgca tcttcattac taaaggcgcc ggggttcagg 1140

gactgcaaaa cggcgcggtg agctgtatcg gcatgaccgg cgctgtgccg tcgggcattc 1200

gggcggtgct ggcggaaaac ctgatcgcct ctatgctcga cctcgaagtg gcgtccgcca 1260

acgaccagac tttctcccac tcggatattc gccgcaccgc gcgcaccctg atgcagatgc 1320

tgccgggcac cgactttatt ttctccggct acagcgcggt gccgaactac gacaacatgt 1380

tcgccggctc gaacttcgat gcggaagatt ttgatgatta caacatcctg cagcgtgacc 1440

tgatggttga cggcggcctg cgtccggtga ccgaggcgga aaccattgcc attcgccaga 1500

aagcggcgcg ggcgatccag gcggttttcc gcgagctggg gctgccgcca atcgccgacg 1560

aggaggtgga ggccgccacc tacgcgcacg gcagcaacga gatgccgccg cgtaacgtgg 1620

tggaggatct gagtgcggtg gaagagatga tgaagcgcaa catcaccggc ctcgatattg 1680

tcggcgcgct gagccgcagc ggctttgagg atatcgccag caatattctc aatatgctgc 1740
gccagcgggt caccggcgat tacctgcaga cctcggccat tctcgatcgg cagttcgagg 1800
tggtgagtgc ggtcaacgac atcaatgact atcaggggcc gggcaccggc tatcgcatct 1860
ctgccgaacg ctgggcggag atcaaaaata ttccgggcgt ggttcagccc gacaccattg 1920
aataaggcgg tattcctgtg caacagacaa cccaaattca gccctctttt accctgaaaa 1980
cccgcgaggg cggggtagct tctgccgatg aacgcgccga tgaagtggtg atcggcgtcg 2040
gccctgcctt cgataaacac cagcatcaca ctctgatcga tatgccccat ggcgcgatcc 2100
tcaaagagct gattgccggg gtggaagaag aggggcttca cgcccgggtg gtgcgcattc 2160
tgcgcacgtc cgacgtctcc tttatggcct gggatgcggc caacctgagc ggctcgggga 2220
tcggcatcgg tatccagtcg aaggggacca cggtcatcca tcagcgcgat ctgctgccgc 2280
tcagcaacct ggagctgttc tcccaggcgc cgctgctgac gctggagacc taccggcaga 2340
ttggcaaaaa cgctgcgcgc tatgcgcgca aagagtcacc ttcgccggtg ccggtggtga 2400
acgatcagat ggtgcggccg aaatttatgg ccaaagccgc gctatttcat atcaaagaga 2460
ccaaacatgt ggtgcaggac gccgagcccg tcaccctgca catcgactta gtaagggagt 2520
gaccatgagc gagaaaacca tgcgcgtgca ggattatccg ttagccaccc gctgcccgga 2580
gcatatcctg acgcctaccg gcaaaccatt gaccgatatt accctcgaga aggtgctctc 2640
tggcgaggtg ggcccgcagg atgtgcggat ctcccgccag acccttgagt accaggcgca 2700
gattgccgag cagatgcagc gccatgcggt ggcgcgcaat ttccgccgcg cggcggagct 2760
tatcgccatt cctgacgagc gcattctggc tatctataac gcgctgcgcc cgttccgctc 2820
ctcgcaggcg gagctgctgg cgatcgccga cgagctggag cacacctggc atgcgacagt 2880
gaatgccgcc tttgtccggg agtcggcgga agtgtatcag cagcggcata agctgcgtaa 2940
aggaagctaa gcggaggtca gcatgccgtt aatagccggg attgataatt ccgggcccgt 3000
cgacagatcc ccgggaattc atcgtgactg actgacgatc tgcctcgcgc gtttcggtga 3060
tgacggtgaa aacctctgac acatgcagct cccggagacg gtcacagctt gtctgtaagc 3120
ggatgccggg agcagacaag cccgtcaggg cgcgtcagcg ggtgttggcg ggtgtcgggg 3180
cgcagccatg acccagtcac gtagcgatag cggagtgtat aattcttgaa gacgaaaggg 3240
cctcgtgata cgcctatttt tataggttaa tgtcatgata ataatggttt cttagacgtc 3300
aggtggcact tttcggggaa atgtgcgcgg aacccctatt tgtttatttt tctaaataca 3360
ttcaaatatg tatccgctca tgagacaata accctgataa atgcttcaat aatattgaaa 3420
aaggaagagt atgagtattc aacatttccg tgtcgccctt attccctttt ttgcggcatt 3480
ttgccttcct gtttttgctc acccagaaac gctggtgaaa gtaaaagatg ctgaagatca 3540
gttgggtgca cgagtgggtt acatcgaact ggatctcaac agcggtaaga tccttgagag 3600
ttttcgcccc gaagaacgtt ttccaatgat gagcactttt aaagttctgc tatgtggcgc 3660
ggtattatcc cgtgttgacg ccgggcaaga gcaactcggt cgccgcatac actattctca 3720

gaatgacttg gttgagtact caccagtcac agaaaagcat cttacggatg gcatgacagt 3780
aagagaatta tgcagtgctg ccataaccat gagtgataac actgcggcca acttacttct 3840
gacaacgatc ggaggaccga aggagctaac cgcttttttg cacaacatgg gggatcatgt 3900
aactcgcctt gatcgttggg aaccggagct gaatgaagcc ataccaaacg acgagcgtga 3960
caccacgatg cctgcagcaa tggcaacaac gttgcgcaaa ctattaactg gcgaactact 4020
tactctagct tcccggcaac aattaataga ctggatggag gcggataaag ttgcaggacc 4080
acttctgcgc tcggcccttc cggctggctg gtttattgct gataaatctg gagccggtga 4140
gcgtgggtct cgcggtatca ttgcagcact ggggccagat ggtaagccct cccgtatcgt 4200
agttatctac acgacgggga gtcaggcaac tatggatgaa cgaaatagac agatcgctga 4260
gataggtgcc tcactgatta agcattggta actgtcagac caagtttact catatatact 4320
ttagattgat ttaaaacttc atttttaatt taaaaggatc taggtgaaga tcctttttga 4380
taatctcatg accaaaatcc cttaacgtga gttttcgttc cactgagcgt cagaccccgt 4440
agaaaagatc aaaggatctt cttgagatcc tttttttctg cgcgtaatct gctgcttgca 4500
aacaaaaaaa ccaccgctac cagcggtggt ttgtttgccg gatcaagagc taccaactct 4560
ttttccgaag gtaactggct tcagcagagc gcagatacca aatactgtcc ttctagtgta 4620
gccgtagtta ggccaccact tcaagaactc tgtagcaccg cctacatacc tcgctctgct 4680
aatcctgtta ccagtggctg ctgccagtgg cgataagtcg tgtcttaccg ggttggactc 4740
aagacgatag ttaccggata aggcgcagcg gtcgggctga acggggggtt cgtgcacaca 4800
gcccagcttg gagcgaacga cctacaccga actgagatac ctacagcgtg agctatgaga 4860
aagcgccacg cttcccgaag ggagaaaggc ggacaggtat ccggtaagcg gcagggtcgg 4920
aacaggagag cgcacgaggg agcttccagg gggaaacgcc tggtatcttt atagtcctgt 4980
cgggtttcgc cacctctgac ttgagcgtcg atttttgtga tgctcgtcag gggggcggag 5040
cctatggaaa aacgccagca acgcggcctt tttacggttc ctggcctttt gctggccttt 5100
tgctcacatg ttctttcctg cgttatcccc tgattctgtg gataaccgta ttaccgcctt 5160
tgagtgagct gataccgctc gccgcagccg aacgaccgag cgcagcgagt cagtgagcga 5220
ggaagcggaa gagcgcctga tgcggtattt tctccttacg catctgtgcg gtatttcaca 5280
ccgcataaat tccgacacca tcgaatggtg caaaaccttt cgcggtatgg catgatagcg 5340
cccggaagag agtcaattca gggtggtgaa tgtgaaacca gtaacgttat acgatgtcgc 5400
agagtatgcc ggtgtctctt atcagaccgt ttcccgcgtg gtgaaccagg ccagccacgt 5460
ttctgcgaaa acgcgggaaa aagtggaagc ggcgatggcg gagctgaatt acattcccaa 5520
ccgcgtggca caacaactgg cgggcaaaca gtcgttgctg attggcgttg ccacctccag 5580
tctggccctg cacgcgccgt cgcaaattgt cgcggcgatt aaatctcgcg ccgatcaact 5640
gggtgccagc gtggtggtgt cgatggtaga acgaagcggc gtcgaagcct gtaaagcggc 5700
ggtgcacaat cttctcgcgc aacgcgtcag tgggctgatc attaactatc cgctggatga 5760
ccaggatgcc attgctgtgg aagctgcctg cactaatgtt ccggcgttat ttcttgatgt 5820

ctctgaccag acacccatca acagtattat tttctcccat gaagacggta cgcgactggg 5880

cgtggagcat ctggtcgcat tgggtcacca gcaaatcgcg ctgttagcgg gcccattaag 5940

ttctgtctcg gcgcgtctgc gtctggctgg ctggcataaa tatctcactc gcaatcaaat 6000

tcagccgata gcggaacggg aaggcgactg gagtgccatg tccggttttc aacaaaccat 6060

gcaaatgctg aatgagggca tcgttcccac tgcgatgctg gttgccaacg atcagatggc 6120

gctgggcgca atgcgcgcca ttaccgagtc cgggctgcgc gttggtgcgg atatctcggt 6180

agtgggatac gacgataccg aagacagctc atgttatatc ccgccgttaa ccaccatcaa 6240

acaggatttt cgcctgctgg ggcaaaccag cgtggaccgc ttgctgcaac tctctcaggg 6300

ccaggcggtg aagggcaatc agctgttgcc cgtctcactg gtgaaaagaa aaaccaccct 6360

ggcgcccaat acgcaaaccg cctctccccg cgcgttggcc gattcattaa tgcagctggc 6420

acgacaggtt tcccgactgg aaagcgggca gtgagcgcaa cgcaattaat gtgagttagc 6480

tcactcatta ggcaccccag gctttacact ttatgcttcc ggctcgtatg ttgtgtggaa 6540

ttgtgagcgg ataacaattt cacacaggaa acagctatga ccatgattac ggattcactg 6600

gccgtcgttt tacaacgtcg tgactgggaa aaccctggcg ttacccaact taatcgcctt 6660

gcagcacatc cccctttcgc cagctggcgt aatagcgaag aggcccgcac cgatcgccct 6720

tcccaacagt tgcgcagcct gaatggcgaa tggcgctttg cctggtttcc ggcaccagaa 6780

gcggtgccgg aaagctggct ggagtgcgat cttcctgagg ccgatactgt cgtcgtcccc 6840

tcaaactggc agatgcacgg ttacgatgcg cccatctaca ccaacgtaac ctatcccatt 6900

acggtcaatc cgccgtttgt tcccacggag aatccgacgg gttgttactc gctcacattt 6960

aatgttgatg aaagctggct acaggaaggc cagacgcgaa ttatttttga tggcgttgga 7020

att 7023

<210> 4
<211> 6608
<212> DNA
<213> plasmid p15A

<400> 4
gaattaattc tggcgaatcc tctgaccagc cagaaaacga cctttctgtg gtgaaaccgg 60

atgctgcaat tcagagcgcc agcaagtggg ggacagcaga agacctgacc gccgcagagt 120

ggatgtttga catggtgaag actatcgcac catcagccag aaaaccgaat tttgctgggt 180

gggctaacga tatccgcctg atgcgtgaac gtgacggacg taaccaccgc gacatgtgtg 240

tgctgttccg ctgggcatgc caggacaact tctggtccgg taacgtgctg agcccggcca 300

agcttactcc ccatccccct gttgacaatt aatcatcggc tcgtataatg tgtggaattg 360

tgagcggata acaatttcac acaggaaaca ggatcctagg aggtttaaac atatgtcgct 420

ttcaccgcca ggcgtacgcc tgttttacga tccgcgcggg caccatgccg gcgccatcaa 480

tgagctgtgc tgggggctgg aggagcaggg ggtcccctgc cagaccataa cctatgacgg 540

aggcggtgac gccgctgcgc tgggcgccct ggcggccaga agctcgcccc tgcgggtggg 600

tatcgggctc agcgcgtccg gcgagatagc cctcactcat gcccagctgc cggcggacgc 660
gccgctggct accggacacg tcaccgatag cgacgatcaa ctgcgtacgc tcggcgccaa 720
cgccgggcag ctggttaaag tcctgccgtt aagtgagaga aactgaagat cctaggaggt 780
ttaaacatat gccgttaata gccgggattg atatcggcaa cgccaccacc gaggtggcgc 840
tggcgtccga ctacccgcag gcgagggcgt ttgttgccag cgggatcgtc gcgacgacgg 900
gcatgaaagg gacgcgggac aatatcgccg ggaccctcgc cgcgctggag caggccctgg 960
cgaaaacacc gtggtcgatg agcgatgtct ctcgcatcta tcttaacgaa gccgcgccgg 1020
tgattggcga tgtggcgatg gagaccatca ccgagaccat tatcaccgaa tcgaccatga 1080
tcggtcataa cccgcagacg ccgggcgggg tgggcgttgg cgtggggacg actatcgccc 1140
tcgggcggct ggcgacgctg ccggcggcgc agtatgccga ggggtggatc gtactgattg 1200
acgacgccgt cgatttcctt gacgccgtgt ggtggctcaa tgaggcgctc gaccggggga 1260
tcaacgtggt ggcggcgatc ctcaaaaagg acgacggcgt gctggtgaac aaccgcctgc 1320
gtaaaaccct gccggtggtg gatgaagtga cgctgctgga gcaggtcccc gagggggtaa 1380
tggcggcggt ggaagtggcc gcgccgggcc aggtggtgcg gatcctgtcg aatccctacg 1440
ggatcgccac cttcttcggg ctaagcccgg aagagaccca ggccatcgtc cccatcgccc 1500
gcgccctgat tggcaaccgt tccgcggtgg tgctcaagac cccgcagggg gatgtgcagt 1560
cgcgggtgat cccggcgggc aacctctaca ttagcggcga aaagcgccgc ggagaggccg 1620
atgtcgccga gggcgcggaa gccatcatgc aggcgatgag cgcctgcgct ccggtacgcg 1680
acatccgcgg cgaaccgggc acccacgccg gcggcatgct tgagcgggtg cgcaaggtaa 1740
tggcgtccct gaccggccat gagatgagcg cgatatacat ccaggatctg ctggcggtgg 1800
atacgtttat tccgcgcaag gtgcagggcg ggatggccgg cgagtgcgcc atggagaatg 1860
ccgtcgggat ggcggcgatg gtgaaagcgg atcgtctgca aatgcaggtt atcgcccgcg 1920
aactgagcgc ccgactgcag accgaggtgg tggtgggcgg cgtggaggcc aacatggcca 1980
tcgccggggc gttaaccact cccggctgtg cggcgccgct ggcgatcctc gacctcggcg 2040
ccggctcgac ggatgcggcg atcgtcaacg cggaggggca gataacggcg gtccatctcg 2100
ccggggcggg gaatatggtc agcctgttga ttaaaaccga gctgggcctc gaggatcttt 2160
cgctggcgga agcgataaaa aaatacccgc tggccaaagt ggaaagcctg ttcagtattc 2220
gtcacgagaa tggcgcggtg gagttctttc gggaagccct cagcccggcg gtgttcgcca 2280
aagtggtgta catcaaggag ggcgaactgg tgccgatcga taacgccagc ccgctggaaa 2340
aaattcgtct cgtgcgccgg caggcgaaag agaaagtgtt tgtcaccaac tgcctgcgcg 2400
cgctgcgcca ggtctcaccc ggcggttcca ttcgcgatat cgcctttgtg gtgctggtgg 2460
gcggctcatc gctggacttt gagatcccgc agcttatcac ggaagccttg tcgcactatg 2520
gcgtggtcgc cgggcagggc aatattcggg gaacagaagg gccgcgcaat gcggtcgcca 2580
ccgggctgct actggccggt caggcgaatt aaagatctcg ggtagcccgc ctaatgagcg 2640

ggcttttttt tatgagaatt acaacttata tcgtatgggg ctgacttcag gtgctacatt 2700
tgaagagata aattgcactg aaatctagaa atattttatc tgattaataa gatgatcttc 2760
ttgagatcgt tttggtctgc gcgtaatctc ttgctctgaa aacgaaaaaa ccgccttgca 2820
gggcggtttt tcgaaggttc tctgagctac caactctttg aaccgaggta actggcttgg 2880
aggagcgcag tcaccaaaac ttgtcctttc agtttagcct taaccggcgc atgacttcaa 2940
gactaactcc tctaaatcaa ttaccagtgg ctgctgccag tggtgctttt gcatgtcttt 3000
ccgggttgga ctcaagacga tagttaccgg ataaggcgca gcggtcggac tgaacggggg 3060
gttcgtgcat acagtccagc ttggagcgaa ctgcctaccc ggaactgagt gtcaggcgtg 3120
gaatgagaca aacgcggcca taacagcgga atgacaccgg taaaccgaaa ggcaggaaca 3180
ggagagcgca cgagggagcc gccaggggga aacgcctggt atctttatag tcctgtcggg 3240
tttcgccacc actgatttga gcgtcagatt tcgtgatgct tgtcaggggg gcggagccta 3300
tggaaaaacg gctttgccgc ggccctctca cttccctgtt aagtatcttc ctggcatctt 3360
ccaggaaatc tccgccccgt tcgtaagcca tttccgctcg ccgcagtcga acgaccgagc 3420
gtagcgagtc agtgagcgag gaagcggaat atatcctgta tcacatattc tgctgacgca 3480
ccggtgcagc cttttttctc ctgccacatg aagcacttca ctgacaccct catcagtgcc 3540
aacatagtaa gccagtatac actccgctag cgctgatgtc cggcggtgct tttgccgtta 3600
cgcaccaccc cgtcagtagc tgaacaggag ggacagctga tagaaacaga agccactgga 3660
gcacctcaaa aacaccatca tacactaaat cagtaagttg gcagcatcac ccgacgcact 3720
ttgcgccgaa taaatacctg tgacggaaga tcacttcgca gaataaataa atcctggtgt 3780
ccctgttgat accgggaagc cctgggccaa cttttggcga aaatgagacg ttgatcggca 3840
cgtaagaggt tccaactttc accataatga aataagatca ctaccgggcg tattttttga 3900
gttatcgaga ttttcaggag ctaaggaagc taaaatggag aaaaaaatca ctggatatac 3960
caccgttgat atatcccaat ggcatcgtaa agaacatttt gaggcatttc agtcagttgc 4020
tcaatgtacc tataaccaga ccgttcagct ggatattacg gcctttttaa agaccgtaaa 4080
gaaaaataag cacaagtttt atccggcctt tattcacatt cttgcccgcc tgatgaatgc 4140
tcatccggaa tttcgtatgg caatgaaaga cggtgagctg gtgatatggg atagtgttca 4200
cccttgttac accgttttcc atgagcaaac tgaaacgttt tcatcgctct ggagtgaata 4260
ccacgacgat ttccggcagt ttctacacat atattcgcaa gatgtggcgt gttacggtga 4320
aaacctggcc tatttcccta aagggtttat tgagaatatg tttttcgtct cagccaatcc 4380
ctgggtgagt ttcaccagtt ttgatttaaa cgtggccaat atggacaact tcttcgcccc 4440
cgttttcacc atgggcaaat attatacgca aggcgacaag gtgctgatgc cgctggcgat 4500
tcaggttcat catgccgtct gtgatggctt ccatgtcggc agaatgctta atgaattaca 4560
acagtactgc gatgagtggc agggcggggc gtaatttttt taaggcagtt attggtgccc 4620
ttaaacgcct ggtgctacgc ctgaataagt gataataagc ggatgaatgg cagaaattcg 4680
aaagcaaatt cgacccggtc gtcggttcag ggcagggtcg ttaaatagcc gcttatgtct 4740

attgctggtt taccggttta ttgactaccg gaagcagtgt gaccgtgtgc ttctcaaatg 4800
cctgaggcca gtttgctcag gctctccccg tggaggtaat aattgacgat atgatcattt 4860
attctgcctc ccaaagcaat tccgacacca tcgaatggtg caaaaccttt cgcggtatgg 4920
catgatagcg cccggaagag agtcaattca gggtggtgaa tgtgaaacca gtaacgttat 4980
acgatgtcgc agagtatgcc ggtgtctctt atcagaccgt ttcccgcgtg gtgaaccagg 5040
ccagccacgt ttctgcgaaa acgcgggaaa aagtggaagc ggcgatggcg gagctgaatt 5100
acattcccaa ccgcgtggca caacaactgg cgggcaaaca gtcgttgctg attggcgttg 5160
ccacctccag tctggccctg cacgcgccgt cgcaaattgt cgcggcgatt aaatctcgcg 5220
ccgatcaact gggtgccagc gtggtggtgt cgatggtaga acgaagcggc gtcgaagcct 5280
gtaaagcggc ggtgcacaat cttctcgcgc aacgcgtcag tgggctgatc attaactatc 5340
cgctggatga ccaggatgcc attgctgtgg aagctgcctg cactaatgtt ccggcgttat 5400
ttcttgatgt ctctgaccag acacccatca acagtattat tttctcccat gaagacggta 5460
cgcgactggg cgtggagcat ctggtcgcat tgggtcacca gcaaatcgcg ctgttagcgg 5520
gcccattaag ttctgtctcg gcgcgtctgc gtctggctgg ctggcataaa tatctcactc 5580
gcaatcaaat tcagccgata gcggaacggg aaggcgactg gagtgccatg tccggttttc 5640
aacaaaccat gcaaatgctg aatgagggca tcgttcccac tgcgatgctg gttgccaacg 5700
atcagatggc gctgggcgca atgcgcgcca ttaccgagtc cgggctgcgc gttggtgcgg 5760
atatctcggt agtgggatac gacgataccg aagacagctc atgttatatc ccgccgttaa 5820
ccaccatcaa acaggatttt cgcctgctgg ggcaaaccag cgtggaccgc ttgctgcaac 5880
tctctcaggg ccaggcggtg aagggcaatc agctgttgcc cgtctcactg gtgaaaagaa 5940
aaaccaccct ggcgcccaat acgcaaaccg cctctccccg cgcgttggcc gattcattaa 6000
tgcagctggc acgacaggtt tcccgactgg aaagcgggca gtgagcgcaa cgcaattaat 6060
gtgagttagc tcactcatta ggcaccccag gctttacact ttatgcttcc ggctcgtatg 6120
ttgtgtggaa ttgtgagcgg ataacaattt cacacaggaa acagctatga ccatgattac 6180
ggattcactg gccgtcgttt tacaacgtcg tgactgggaa aaccctggcg ttacccaact 6240
taatcgcctt gcagcacatc cccctttcgc cagctggcgt aatagcgaag aggcccgcac 6300
cgatcgccct tcccaacagt tgcgcagcct gaatggcgaa tggcgctttg cctggtttcc 6360
ggcaccagaa gcggtgccgg aaagctggct ggagtgcgat cttcctgagg ccgatactgt 6420
cgtcgtcccc tcaaactggc agatgcacgg ttacgatgcg cccatctaca ccaacgtaac 6480
ctatcccatt acggtcaatc cgccgtttgt tcccacggag aatccgacgg gttgttactc 6540
gctcacattt aatgttgatg aaagctggct acaggaaggc cagacgcgaa ttatttttga 6600
tggcgttg 6608